(19) Europäisches Patentamt
European Patent Office
Office européen des brevets

(11) EP 1 859 793 B1

(12) EUROPEAN PATENT SPECIFICATION

(45) Date of publication and mention of the grant of the patent:
**20.04.2011 Bulletin 2011/16**

(21) Application number: **06715261.1**

(22) Date of filing: **28.02.2006**

(51) Int Cl.:
***A61K 31/18*** (2006.01) ***A61K 31/343*** (2006.01)
***A61K 31/381*** (2006.01) ***A61K 31/404*** (2006.01)
***A61K 31/498*** (2006.01) ***A61K 31/517*** (2006.01)
***A61K 31/64*** (2006.01) ***A61K 39/395*** (2006.01)
***A61K 45/00*** (2006.01) ***A61P 35/00*** (2006.01)
***A61P 43/00*** (2006.01)

(86) International application number:
**PCT/JP2006/304218**

(87) International publication number:
**WO 2006/090930 (31.08.2006 Gazette 2006/35)**

(54) **NOVEL COMBINATIONAL USE OF A SULFONAMIDE COMPOUND IN THE TREATMENT OF CANCER**

NEUE KOMBINIERTE ANWENDUNG EINER SULFONAMID-VERBINDUNG ZUR BEHANDLUNG VON KREBS

NOUVELLE UTILISATION COMBINEE D'UN COMPOSE DE SULFONAMIDE DANS LE TRAITMENT DU CANCER

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.02.2005 JP 2005054111**

(43) Date of publication of application:
**28.11.2007 Bulletin 2007/48**

(73) Proprietor: **Eisai R&D Management Co., Ltd.**
**Tokyo 112-8088 (JP)**

(72) Inventors:
- **OWA, Takashi,**
**Tsukuba Res. Lab., Eisai Co., Ltd.**
**Tsukuba-shi,Ibaraki 3002635 (JP)**
- **OZAWA, Yoichi,**
**Tsukuba Res. Lab., Eisai Co., Ltd.**
**Tsukuba-shi,Ibaraki 3002635 (JP)**
- **SEMBA, Taro,**
**Tsukuba Res. Lab., Eisai Co., Ltd.**
**Tsukuba-shi,Ibaraki 3002635 (JP)**
- **WAKABAYASHI, T.,**
**Tsukuba Res. Lab., Eisai Co., Ltd**
**Tsukuba-shi,Ibaraki 3002635 (JP)**

(74) Representative: **Woods, Geoffrey Corlett**
**J.A. Kemp & Co.**
**14 South Square**
**Gray's Inn**
**London**
**WC1R 5JJ (GB)**

(56) References cited:
**EP-A- 0 673 937**
**EP-A- 1 481 678**
**WO-A-01/97843**
**WO-A-2004/026872**
**WO-A1-03/035629**
**WO-A1-03/074045**
**WO-A1-2006/030947**
**JP-A- 07 165 708**
**JP-A- 62 096 459**
**JP-A- 2002 114 710**
**JP-A- 2004 530 709**
**JP-A- 2004 536 129**
**US-A1- 2004 209 930**

- **SHIMIZU K ET AL: "Cancer anti-angiogenic therapy" BIOLOGICAL AND PHARMACEUTICAL BULLETIN 200405 JP, vol. 27, no. 5, May 2004 (2004-05), pages 599-605, XP002483110 ISSN: 0918-6158 1347-5215**
- **ARAO T ET AL: "Small in-frame deletion in the epidermal growth factor receptor as a target for ZD6474" CANCER RESEARCH 20041215 US, vol. 64, no. 24, 15 December 2004 (2004-12-15), pages 9101-9104, XP002483111 ISSN: 0008-5472**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- FUKUOKA K ET AL: "Mechanisms of action of the novel sulfonamide anticancer agent E7070 on cell cycle progression in human non-small cell lung cancer cells" INVESTIGATIONAL NEW DRUGS 2001 US, vol. 19, no. 3, 2001, pages 219-227, XP002483112 ISSN: 0167-6997
- MAZITSCHEK R. ET AL.: 'Inhibitors of angiogenesis and Cancer-related receptor tyrosine kinases' CURRENT OPINION IN CHEMICAL BIOLOGY vol. 8, 2004, pages 432 - 441, XP003002235
- YANO S. ET AL.: 'EGFR tyrosine kinase inhibitor "gefitinib(Iressa)" for cancer therapy' NIPPON YAKURIGAKU ZASSHI vol. 122, 2003, pages 491 - 497, XP003002236

## Description

FIELD OF THE INVENTION

The present invention relates to a novel pharmaceutical composition, a kit and their use for treating cancer.

BACKGROUND OF THE INVENTION

Examples of conventionally used chemotherapy drugs for cancer include alkylating agents such as cyclophosphamide, antimetabolites such as methotrexate and fluorouracil, antibiotics such as adriamycin, mitomycin, bleomycin, plant-derived taxol, vincristine and etoposide, and metal complexes such as cisplatin. All of them, however, have not been sufficient in anti-tumor effects, and thus there has been a strong need for development of a novel anti-tumor agent.

Recently, a sulfonamide compound has been reported as a useful anti-tumor agent [1-5]. In particular, N-(3-chloro-1H-indole-7-yl)-4-sulfamoylbenzenesulfonamide (hereinafter, also referred to as "E7070"), N-(3-cyano-4-methyl-1H-indole-7-yl)-3-cyanobenzenesulfonamide (hereinafter, also referred to as "E7820"), N-[[(4-chlorophenyl)amino]carbonyl]-2,3-dihydro- 1H-indene-5-sulfonamide (hereinafter, also referred to as "LY186641"), N-[[(3,4-dichlorophenyl)amino]carbonyl]-2,3-dihydrobenzofuran-5-sulfonamide (hereinafter, also referred to as "LY295501"), N-(2,4 dichlorobenzoyl)-4-chlorophenylsulfonamide (hereinafter, also referred to as "LY-ASAP"), N-(2,4-dichlorobenzoyl)-5-bromothiophene-2-sulfonamide (hereinafter, also referred to as "LY573636") and 2-sulfanylamide-5-chloroquinoxaline (hereinafter, also referred to as "CQS") are active against various types of tumors and thus are very useful.
On the other hand, as substances having an EGF inhibitory activity, EGFR kinase inhibitors 4-(3-chloro-4-fluorophenylamino)-7-methoxy-6-(3-(4-morpholino) propoxy-quinazoline) (hereinafter, also referred to as a "gefitinib") and 4-(3-ethynylphenylamino)-6,7-bis(2-methoxyethoxy)-quinazoline (hereinafter, also referred to as "erlotinib"), and an anti-EGFR antibody cetuximab have been reported[6-9].

The presence and the kind of effects resulting from combining these compounds, however, have not been reported so far.

Recently, methods were established for simultaneously detecting expression levels of multiple genes using various DNA microarrays. Thus, DNA microarrays have been used for wide-ranging purposes [10 and 11]. In addition, several reports have been made about using DNA microarrays (In part, there is a macroarray using membrane filters) for examining changes in gene expressions upon use of anti-cancer drugs against tumor cells[12-14]. These reports show that the analysis of gene expression variability is highly useful in comprehensively studying the characteristic comparison among a plurality of cell populations, the biological changes in cells caused by treatment of drug or the like at molecular level.

Furthermore, reports have also been made to the analysis of gene expression profiles of 60 types of cancer cell line panels from the US National Cancer Institute for reclassification of these cell lines and examination of their characteristics[15], and to discussion regarding relationship among the gene expression profiles of these 60 types of cancer cell line panels and sensitivity of each cell line to various anti-cancer drugs[16].

References

(1) Japanese Laid-Open Patent Publication No. 7-165708.
(2) International Publication No. WO00/50395.
(3) European Patent Publication No. 0222475.
(4) International Publication No. WO02/098848.
(5) International Publication No. WO2003/035629.
(6) International Publication No. WO96/33980.
(7) Japanese Patent No. 3040486.
(8) Japanese Patent No. 3088018.
(9) Japanese Laid-Open Patent Publication No. 2-291295.
(10) Schena M, Shalon D, Davis RW, Brown PO. Science, 1995, 270, 467-70.
(11) Lockhart, D.J., Dong, H., Byrne, M.C., Follettie, M.T., Gallo, M.V., Chee, M.S., Mittmann, M., Wang C., Kobayashi, M., Horton, H. Brown, E.L., Nature Biotechnology, 1996, 14, 1675-1680.
(12) Rhee CH, Ruan S, Chen S, Chenchik A, Levin VA, Yung AW, Fuller GN, Zhang W, Oncol Rep, 1999, 6, 393-401.
(13) Zimmermann J, Erdmann D, Lalande I, Grossenbacher R, Noorani M, Furst P, Oncogene, 2000, 19, 2913-20.
(14) Kudoh K, Ramanna M, Ravatn R, Elkahloun AG, Bittner ML, Meltzer PS, Trent JM, Dalton WS, Chin KV, Cancer Res, 2000, 4161-6.

(15) Ross DT, Scherf U, Eisen MB, Perou CM, Rees C, Spellman P, Iyer V, Jeffrey SS, Van de Rijn M, Waltham M, Pergamenschikov A, Lee JC, Lashkari D, Shalon D, Myers TG, Weinstein JN, Botstein D, Brown PO, Nat Genet, 2000, 24, 227-35.

(16) Scherf U, Ross DT, Waltham M, Smith LH, Lee JK, Tanabe L, Kohn KW, Reinhold WC, Myers TG, Andrews DT, Scudiero DA, Eisen MB, Sausville EA, Pommier Y, Botstein D, Brown PO, Weinstein JN, Nat Genet, 2000, 24, 236-44.

DISCLOSURE OF THE INVENTION

The present invention was achieved regarding the circumstances described above. The problem to be solved by the invention is to find a pharmaceutical composition and a kit having a remarkable anti-tumor activity, and a pharmaceutical composition or kit for use in treating cancer.

In order to solve the above problem, the present inventors have gone through keen examination, as a result of which combinational use of E7820 and gefitinib was found to show a statistically significant (by Isobologram) synergistic antiproliferative effect in a cell proliferation assay (*in vitro*). In addition, combinational use of E7820 and gefitinib or erlotinib was found to show a statistically significant (by two-way ANOVA) synergistic anti-tumor effect in a subcutaneous transplant model *(in vivo)* of human non-small-cell lung cancer cell line. Moreover, combinational use of E7820 and gefitinib or erlotinib showed a remarkable anti-tumor effect that cannot be seen with gefitinib or erlotinib alone. The combinational use of E7820 and cetuximab was found to show a remarkable anti-tumor effect.

Furthermore, combinational use of E7070 and gefitinib or erlotinib was found to show a statistically significant (by Isobologram) synergistic antiproliferative effect.

In experiments using DNA microarrays and cancer cell line panels, genetic alteration patterns and antiproliferative activities of E7070, E7820, LY186641, LY295501, LY573636, CQS and combinations thereof were found to show high correlation. In an assay for determining antiproliferative activity, a cancer cell line resistant to E7070 was found to show cross-resistance to E7820, LY186641, LY295501, LY-ASAP, LY573636 or CQS. From these results, the present inventors have found that E7070, E7820, LY186641, LY295501, LY-ASAP, LY573636, CQS and combinations thereof have the same or similar action mechanisms that result in the same or similar gene alterations and effects.

Accordingly, E7070, E7820, or a combination thereof is considered to show a good anti-tumor activity when used in combination with a substance having an EGF inhibitory activity. Thus E7070, E7820, or a combination thereof, and a substance having an EGF inhibitory activity selected from gefitinib, erlotinib or cetuximab, can be used as a useful pharmaceutical composition or a kit, which can be used for treatment of cancer.

Thus, the present invention relates to:

(1) A pharmaceutical composition comprising a sulfonamide compound in combination with a substance having an EGF inhibitory activity, wherein the sulfonamide compound is:

N-(3-chloro-1H-indole-7-yl)-4-sulfamoylbenzenesulfonamide, N-(3-cyano-4-methyl-1H-indole-7-yl)-3-cyanobenzenesulfonamide, or a pharmacologically acceptable salt thereof or a solvate thereof and wherein the substance having an EGF inhibitory activity is gefitinib, erlotinib or cetuximab.

(2) A kit comprising a set of a formulation comprising a sulfonamide compound as defined in (1) and a formulation comprising a substance having an EGF inhibitory activity as defined in (1).

(3) Use of a sulfonamide compound as defined in (1) for producing a pharmaceutical composition for use in combination with a substance having an EGF inhibitory activity as defined in (1) in the treatment of cancer.

(4) Use of a substance having an EGF inhibitory activity as defined in (1) for producing a pharmaceutical composition for use in combination with a sulfonamide compound as defined in (1) in the treatment of cancer.

(5) A sulfonamide compound as defined in (1) for use in combination with a substance having an EGF inhibitory activity as defined in (1) in the treatment of cancer.

(6) A substance having an EGF inhibitory activity as defined in (1) for use in combination with a sulfonamide compound as defined in (1) in the treatment of cancer.

(7) A pharmaceutical composition comprising a sulfonamide compound as defined in (1) for use in combination with a substance having an EGF inhibitory activity as defined in (1) in the treatment of cancer.

(8) A pharmaceutical composition comprising a substance having an EGF inhibitory activity as defined in claim 1 for use in combination with a sulfonamide compound as defined in claim 1 in the treatment of cancer.

The sulfonamide compounds according to (1)-(8) above include at least one compound selected from the group consisting of:

N-(3-chloro- H-indole-7-yl)-4-sulfamoylbenzenesulfonamide,
N-(3-cyano-4-methyl-1H-indole-7-yl)-3-cyanobenzenesulfonamide,

or a pharmacologically acceptable salt thereof, or a solvate thereof.

In (1)-(8) above, the substance having an EGF inhibitory activity may be an EGF receptor kinase inhibitor or an anti-EGFR antibody.

The EGF receptor kinase inhibitor is at least one compound selected from the group consisting of:

4-(3-chloro-4-fluorophenylamino)-7-methoxy-6-(3-(4-morpholino)propoxy-quinazoline),
4-(3-ethynylphenylamino)-6,7-bis(2-methoxyethoxy)-quinazoline;

or a pharmacologically acceptable salt thereof or a solvate thereof.

The anti-EGFR antibody is cetuximab.

The present invention provides a pharmaceutical composition and a kit that show a remarkable anti-tumor activity.

More specifically, the present invention provides a pharmaceutical composition and a kit that show a remarkable anti-tumor activity, by combining a sulfonamide compound, that is, at least one compound selected from E7070 or E7820, with a substance having an EGF inhibitory activity, being at least one selected from gefitinib, erlotinib and cetuximab. Thus, the pharmaceutical composition and the kit of the invention can be used for cancer treatment.

BRIEF DESCRIPTION OF THE DRAWINGS

Figure 1 is a theoretical view of Isobologram method.

Figure 2 shows the effect of combinational use of E7820 and gefitinib in a cell proliferation assay according to Isobologram method.

Figure 3 shows the effect of combinational use of E7070 and gefitinib in a cell proliferation assay according to Isobologram method.

Figure 4 shows the effect obtained by combinational use of E7070 and erlotinib in a cell proliferation assay according to Isobologram method.

Figure 5 shows the effect obtained by combinational use of E7820 and gefitinib in a subcutaneous transplant model of human non-small-cell lung cancer cell line (PC9). In the figure, * indicates a statistically significant synergistic effect at a significance level of less than 0.01. In the figure, Day# indicates days from the first day of administration (Day 1).

Figure 6 shows the effect obtained by combinational use of E7820 and gefitinib in a subcutaneous transplant model of human non-small-cell lung cancer cell line (A549). In the figure, * indicates a statistically significant synergistic effect at a significance level of less than 0.01. In the figure, Day# indicates days from the first day of administration (Day 1).

Figure 7 shows the effect obtained by combinational use of E7820 and erlotinib in a subcutaneous transplant model of human non-small-cell lung cancer cell line (A549). In the figure, * indicates a statistically significant synergistic effect at a significance level of less than 0.01. In the figure, Day# indicates days from the first day of administration (Day 1).

Figure 8 shows the results of hierarchical cluster analysis in the DNA microarrays in Example 7.

Figure 9 shows correlation coefficients in the DNA microarrays in Example 8.

Figure 10 shows the results of hierarchical cluster analysis in the DNA microarrays in Example 8.

Figure 11 shows correlation coefficients in the DNA microarrays in Example 8.

Figure 12 shows the results of hierarchical cluster analysis in the DNA microarrays in Example 8.

Figure 13 shows antiproliferative effects of E7070, E7820, CQS, LY186641, LY295501 and LY-ASAP on HCT116-C9, HCT116-C9-C1 and HCT116-C9-C4 as measured by cell growth inhibition assay.

Figure 14 shows antiproliferative effects of E7070 and LY573636 on

HCT116-C9, HCT116-C9-C1 and HCT116-C9-C4 as measured by cell growth inhibition assay.

BEST MODES FOR CARRYING OUT THE INVENTION

Hereinafter, embodiments of the present invention will be described. The following embodiments are described for illustrating the present invention and they are not intended to limit the present invention. The present invention may

be carried out in various embodiments as long as it does not depart from the scope of the invention.

The publications, laid-open patent publications, patent publications and other patent documents cited herein are incorporated herein by reference.

1. Sulfonamide compound

A pharmaceutical composition and/or a kit of the present invention comprise a sulfonamide compound. According to the present invention, the sulfonamide compound comprises N-(3-chloro-1H-indole-7-yl)-4-sulfamoylbenzenesulfonamide,

N-(3-cyano-4-methyl-1H-indole-7-yl)-3-cyanobenzenesulfonamide, or a pharmaceutically acceptable salt or solvate thereof.

The sulfonamide compound is E7070 or E7820.

E7070 is N-(3-chloro-1H-indole-7-yl)-4-sulfamoylbenzenesulfonamide, whose structural formula is represented by the following Formula (VIII).

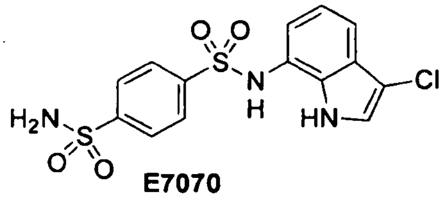

(VIII)

E7070 can be produced according to a known method, for example, by those described in International Publication No. 95/07276 (pamphlet) (WO95/07276) and/or Example 19 of Japanese Laid-Open Patent Publication No. 7-165708 (JP7-165708).

E7820 is N-(3-cyano-4-methyl-1H-indole-7-yl)-3-cyanobenzenesulfonamide, whose structural formula is represented by the following Formula (IX).

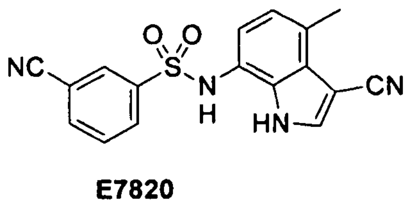

(IX)

E7820

E7820 can be produced according to a known method, for example, by a method described in International Publication No. 00/50395 (pamphlet) (WO00/50395).

The sulfonamide compound may form a pharmacologically acceptable salt with acid or base. The sulfonamide compound of the invention also comprises these pharmacologically acceptable salts. Examples of salts formed with acids include inorganic acid salts such as hydrochloride salts, hydrobromide salts, sulfate salts and phosphate salts, and salts formed with organic acids such as formic acid, acetic acid, lactic acid, succinic acid, fumaric acid, maleic acid, citric acid, tartaric acid, benzoic acid, methanesulfonic acid, benzenesulfonic acid, p-toluenesulfonic acid and trifluoroacetic acid. Examples of salts formed with bases include alkali metal salts such as sodium salt and potassium salt, alkaline earth metal salts such as calcium salt and magnesium salt, salts with organic bases such as trimethylamine, triethylamine, pyridine, picoline, dicyclohexylamine, N,N'-dibenzylethylenediamine, arginine and lysine (organic amine salts), and ammonium salts.

Furthermore, the sulfonamide compound may be in anhydride form, and may form a solvate such as a hydrate. The solvate may be either a hydrate or a nonhydrate, preferably a hydrate. The solvent used may be water, alcohol (e.g., methanol, ethanol or n-propanol), dimethylformamide or the like.

If solvates and/or enantiomers of these compounds exist, the sulfonamide compound of the invention comprises these solvates and/or enantiomers. The sulfonamide compound of the invention also comprises a sulfonamide compound that undergoes metabolism such as oxidation, reduction, hydrolysis and conjugation *in vivo.* Moreover, the sulfonamide compound of the invention also comprises compounds that generate a sulfonamide compound by undergoing metabolism such as oxidation, reduction and hydrolysis *in vivo.*

2. Substance having EGF inhibitory activity

A pharmaceutical composition and/or a kit of the invention comprise a substance having an EGF inhibitory activity. According to the present invention, the substance having an EGF inhibitory activity inhibits EGF action, activity or the like, and can be an EGF Receptor (EGFR) kinase inhibitor or an anti-EGF Receptor (EGFR) antibody.
EGF inhibitory activity refers to activity in inhibition of physiological activity and/or pharmacological activity of EGF. EGF inhibitory activity may be determined by an existing method, for example, cell proliferation assay, kinase assay or western blotting. The substance may be assumed to be a substance having EGF inhibitory activity when an EGF inhibitory activity quantified by these methods is, for example, 30 μM or lower, preferably 10 μM or lower, more preferably 3 μM or lower, still more preferably 1 μM or lower at 50% inhibitory concentration.

(1) EGFR kinase inhibitor

According to the present invention, an EGFR kinase inhibitor can be gefitinib or erlotinib.
The EGFR kinase inhibitor can be produced by a known method, for example, those that are described in International Publication No. 96/33980 (pamphlet) (WO96/33980), Japanese Patent No. 3040486 (JP3040486) and US Patent No. 5770599 (specification) (US5770599).
A particularly preferable compound is gefitinib.
Gefitinib is
4-(3-chloro-4-fluorophenylamino)-7-methoxy-6-(3-(4-morpholino)propoxy-quinazoline), whose structural formula is represented by the following Formula (XIII).

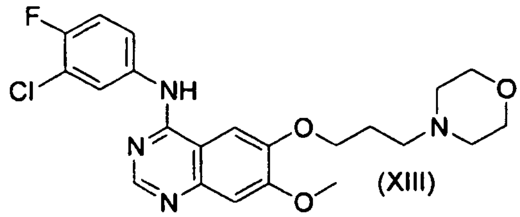
(XIII)

Gefitinib can be produced according to a known method, for example, by those described in International Publication No. 96/33980 (pamphlet) (WO96/33980), Japanese Patent No. 3040486 (JP3040486) and US Patent No. 5770599 (specification) (US5770599).
Also, gefitinib can be obtained by purchasing Iressa® from AstraZeneca.
A particularly preferable compound is erlotinib.
Erlotinib refers to
4-(3-ethynylphenylamino)-6,7-bis(2-methoxyethoxy)-quinazoline, whose structural formula is represented by the following Formula (XIV).

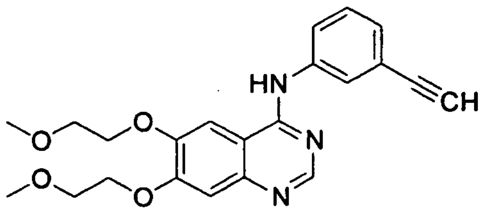
(XIV)

Erlotinib can be produced according to a known method, for example, by those described in International Publication No. 96/30347 (pamphlet) (WO96/30347), Japanese Patent No. 3088018 (JP3088018) and Japanese Patent No. 3420549 (JP3420549).
Erlotinib can also be obtained by purchasing Tarceva® from Genentech.

(2) Anti-EGFR antibody

According to the present invention, the anti-EGFR antibody is cetuximab.

Cetuximab can be obtained according to methods described in Japanese Laid-Open Patent Publication No. 2002-114710 (JP2004-114710) and Japanese Laid-Open Patent Publication No. 2-291295 (JP2-291295).

Moreover, cetuximab can be obtained by purchasing Erbitux® from Merck and Bristol-Myers Squibb.

(3) Salts, hydrates and solvates

A substance having an EGF inhibitory activity may form a pharmacologically acceptable salt with acid or base. A substance having an EGF inhibitory activity of the invention also comprises these pharmacologically acceptable salts. Examples of salts formed with acids include inorganic acid salts such as hydrochloride salts, hydrobromide salts, sulfate salts and phosphate salts, and salts formed with organic acids such as formic acid, acetic acid, lactic acid, succinic acid, fumaric acid, maleic acid, citric acid, tartaric acid, benzoic acid, methanesulfonic acid, benzenesulfonic acid, p-toluenesulfonic acid and trifluoroacetic acid. Examples of salts formed with bases include alkali metal salts such as sodium salt and potassium salt, alkaline earth metal salts such as calcium salt and magnesium salt, and salts formed with organic bases such as trimethylamine, triethylamine, pyridine, picoline, dicyclohexylamine and N,N'-dibenzylethylenediamine, arginine, lysine (organic amine salts), and ammonium salts.

A substance having an EGF inhibitory activity may be an anhydride or may form a solvate such as a hydrate. The solvate may be either a hydrate or a nonhydrate, preferably a hydrate. The solvent may use water, alcohol (e.g., methanol, ethanol or n-propanol), dimethylformamide or the like.

If solvates and/or enantiomers of these substances having an EGF inhibitory activity exist, they are also comprised in the substance having an EGF inhibitory activity of the invention. The substance having an EGF inhibitory activity of the invention also comprises substances having an EGF inhibitory activity that undergo metabolism *in vivo* such as oxidation, reduction, hydrolysis and conjugation. Moreover, the substance having an EGF inhibitory activity of the invention also comprises compounds that generate substances having an EGF inhibitory activity by undergoing metabolism *in vivo* such as oxidation, reduction and hydrolysis.

3. Pharmaceutical composition, kit and method for treating cancer

The present invention relates to a pharmaceutical composition, a kit and a kit or composition for treating cancer, characterized by comprising a sulfonamide compound in combination with a substance having an EGF inhibitory activity.

According to the present invention, a sulfonamide compound is as described in "1. Sulfonamide compound". The sulfonamide compound is at least one compound selected from: E7070 and E7820.

According to the present invention, a substance having an EGF inhibitory activity is as described in "2. Substance having EGF inhibitory activity". The substance having an EGF inhibitory activity is at least one substance selected from: (A) an EGF receptor kinase inhibitor, which is gefitinib or erlotinib; and (B) an anti-EGFR antibody which is cetuximab, i.e. the substance having an EGF inhibitory activity is at least one substance selected from gefitinib, erlotinib and cetuximab.

According to the present invention, the sulfonamide compound and the substance having an EGF inhibitory activity also comprise pharmacologically acceptable salts thereof, or solvates such as hydrates thereof.

According to the present invention, the sulfonamide compound and the substance having an EGF inhibitory activity may be used in any combination.

The pharmaceutical composition of the invention comprises a sulfonamide compound in combination with a substance having an EGF inhibitory activity. The pharmaceutical composition of the invention is useful for treating cancer.

According to the present invention, the term "in combination with" refers to a combination of compounds for combinational use, and includes both modes in which separate compounds are administered in combination and as a mixture.

The pharmaceutical composition of the invention is also provided in another embodiment of a pharmaceutical composition comprising a sulfonamide compound, which is administered to a patient in combination with a substance having an EGF inhibitory activity. The sulfonamide compound and the substance having an EGF inhibitory activity may be administered either simultaneously or separately. The term "simultaneous" refers to administrations at the same timing in a single administration schedule. In this case, it is not necessary to use completely the same hour and minute for administration. The term "separately" refers to administrations at different timings in a single administration schedule.

The kit of the invention comprises a set of a formulation comprising a sulfonamide compound and a formulation comprising a substance having an EGF inhibitory activity. The formulations comprised in the kit of the invention are not limited to a particular form as long as they comprise a sulfonamide compound or a substance having an EGF inhibitory activity. The kit of the invention is useful for treating cancer.

In the kit of the invention, the formulation comprising a sulfonamide compound and the formulation comprising a substance having an EGF inhibitory activity may be mixed together or separately accommodated in a single package. They may be administered simultaneously or one may be administered preceding the other.

The pharmaceutical composition and/or the kit of the invention may be further combined with one or more additional anti-cancer drugs. The additional anti-cancer drugs are not particularly limited as long as they are formulations having an anti-tumor activity. Examples of the additional anti-cancer drugs include irinotecan hydrochloride (CPT-11), oxaliplatin, 5-fluorouracil (5-FU), docetaxel (Taxotere®), gemcitabine hydrochloride (Gemzar®), calcium folinate (Leucovorin) and bevacizumab (Avastin®). Particularly preferable additional anti-cancer drugs are irinotecan hydrochloride, oxaliplatin, 5-fluorouracil, calcium folinate or bevacizumab when the type of cancer to be treated by the drug is colon cancer, gemcitabine hydrochloride or bevacizumab for pancreas cancer, bevacizumab for renal cancer, and docetaxel for lung cancer.

More examples of particularly preferable combinations of the compounds according to the invention are shown in Tables 1, 2, 3 and 4 for the cases of treating colon cancer, pancreas cancer, renal cancer and lung cancer by the therapeutic drug, respectively.

Table 1

| | Combined Compounds | | | | | |
|---|---|---|---|---|---|---|
| 1 | E7070 | Gefitinib | 5-FU | LV | Oxaliplatin | |
| 2 | E7820 | Gefitinib | 5-FU | LV | Oxaliplatin | |
| 3 | E7070 | Erlotinib | 5-FU | LV | Oxaliplatin | |
| 4 | E7820 | Erlotinib | 5-FU | LV | Oxaliplatin | |
| 5 | E7070 | Cetuximab | 5-FU | LV | Oxaliplatin | |
| 6 | E7820 | Cetuximab | 5-FU | LV | Oxaliplatin | |
| 7 | E7070 | Gefitinib | 5-FU | LV | Oxaliplatin | Bevacizumab |
| 8 | E7820 | Gefitinib | 5-FU | LV | Oxaliplatin | Bevacizumab |
| 9 | E7070 | Erlotinib | 5-FU | LV | Oxaliplatin | Bevacizumab |
| 10 | E7820 | Erlotinib | 5-FU | LV | Oxaliplatin | Bevacizumab |
| 11 | E7070 | Cetuximab | 5-FU | LV | Oxaliplatin | Bevacizumab |
| 12 | E7820 | Cetuximab | 5-FU | LV | Oxaliplatin | Bevacizumab |
| 13 | E7070 | Gefitinib | 5-FU | LV | CPT-11 | |
| 14 | E7820 | Gefitinib | 5-FU | LV | CPT-11 | |
| 15 | E7070 | Erlotinib | 5-FU | LV | CPT-11 | |
| 16 | E7820 | Erlotinib | 5-FU | LV | CPT-11 | |
| 17 | E7070 | Cetuximab | 5-FU | LV | CPT-11 | |
| 18 | E7820 | Cetuximab | 5-FU | LV | CPT-11 | |
| 19 | E7070 | Gefitinib | 5-FU | LV | CPT-11 | Bevacizumab |
| 20 | E7820 | Gefitinib | 5-FU | LV | CPT-11 | Bevacizumab |
| 21 | E7070 | Erlotinib | 5-FU | LV | CPT-11 | Bevacizumab |
| 22 | E7820 | Erlotinib | 5-FU | LV | CPT-11 | Bevacizumab |
| 23 | E7070 | Cetuximab | 5-FU | LV | CPT-11 | Bevacizumab |
| 24 | E7820 | Cetuximab | 5-FU | LV | CPT-11 | Bevacizumab |
| 25 | E7070 | Gefitinib | Bevacizumab | | | |
| 26 | E7820 | Gefitinib | Bevacizumab | | | |
| 27 | E7070 | Erlotinib | Bevacizumab | | | |
| 28 | E7820 | Erlotinib | Bevacizumab | | | |
| 29 | E7070 | Cetuximab | Bevacizumab | | | |
| 30 | E7820 | Cetuximab | Bevacizumab | | | |

Table 1 shows preferable combinations of the invention where the type of cancer to be treated by the therapeutic drug for cancer is colon cancer. In the table, LV represents calcium folinate.

Table 2

| | Combined Compounds | | | |
|---|---|---|---|---|
| 1 | E7070 | Gefitinib | Gemcitabine | |
| 2 | E7820 | Gefitinib | Gemcitabine | |
| 3 | E7070 | Erlotinib | Gemcitabine | |
| 4 | E7820 | Erlotinib | Gemcitabine | |
| 5 | E7070 | Cetuximab | Gemcitabine | |
| 6 | E7820 | Cetuximab | Gemcitabine | |
| 7 | E7070 | Gefitinib | Gemcitabine | Bevacizumab |
| 8 | E7820 | Gefitinib | Gemcitabine | Bevacizumab |
| 9 | E7070 | Erlotinib | Gemcitabine | Bevacizumab |
| 10 | E7820 | Erlotinib | Gemcitabine | Bevacizumab |
| 11 | E7070 | Cetuximab | Gemcitabine | Bevacizumab |
| 12 | E7820 | Cetuximab | Gemcitabine | Bevacizumab |

Table 2 shows preferable combinations of the invention where the type of cancer to be treated by the therapeutic drug for cancer is pancreas cancer. In the table, Gemcitabine represents gemcitabine hydrochloride.

Table 3

| | Combined Compounds | | |
|---|---|---|---|
| 1 | E7070 | Gefitinib | Bevacizumab |
| 2 | E7820 | Gefitinib | Bevacizumab |
| 3 | E7070 | Erlotinib | Bevacizumab |
| 4 | E7820 | Erlotinib | Bevacizumab |
| 5 | E7070 | Cetuximab | Bevacizumab |
| 6 | E7820 | Cetuximab | Bevacizumab |

Table 3 shows preferable combinations of the invention where the type of cancer to be treated by the therapeutic drug for cancer is renal cancer.

Table 4

| | Combined Compounds | | |
|---|---|---|---|
| 1 | E7070 | Gefitinib | Docetaxel |
| 2 | E7820 | Gefitinib | Docetaxel |
| 3 | E7070 | Erlotinib | Docetaxel |
| 4 | E7820 | Erlotinib | Docetaxel |
| 5 | E7070 | Cetuximab | Docetaxel |
| 6 | E7820 | Cetuximab | Docetaxel |

Table 4 shows preferable combinations of the invention where the type of cancer to be treated by the therapeutic drug for cancer is lung cancer.

The pharmaceutical composition and/or the kit of the invention can be used as a therapeutic drug for cancer.

Treatments according to the present invention comprise symptomatic relief of the disease, progression delay of symptoms of the disease, elimination of the symptoms of the disease, improvement of prognosis of the disease, and prevention of recurrence of the disease.

A therapeutic drug for cancer according to the invention comprises those that contain an anti-tumor agent, a drug for improving prognosis of cancer, a drug for preventing cancer recurrence, an antimetastatic drug or the like.

The effect of cancer treatment can be confirmed by observation of X-ray pictures, CT or the like, histopathologic diagnosis by biopsy, or tumor marker value.

The pharmaceutical composition and/or the kit of the invention can be administered to mammals (e.g., human, rat, rabbit, sheep, pig, cattle, cat, dog and monkey).

Examples of the types of cancers targeted by the therapeutic drug for cancer include but not limited to at least one selected from the group consisting of brain tumor, cervical cancer, esophageal cancer, tongue cancer, lung cancer, breast cancer, pancreas cancer, gastric cancer, small intestinal and duodenal cancer, colon cancer (colon cancer and rectal cancer), bladder cancer, renal cancer, liver cancer, prostate cancer, uterine cancer, ovarian cancer, thyroid grand cancer, gallbladder cancer, pharyngeal cancer, sarcoma (e.g., osteosarcoma, chondrosarcoma, Kaposi's sarcoma, myosarcoma, angiosarcoma, fibrosarcoma, etc.), leukemia (e.g., chronic myelocytic leukemia (CML), acute myelocytic leukemia (AML), chronic lymphocytic leukemia (CLL), acute lymphocytic leukemia (ALL), lymphoma, multiple myeloma (MM), etc.) and melanoma. Preferably, the type of cancer targeted by the therapeutic drug for cancer is at least one selected from the group consisting of colon cancer, pancreas cancer, renal cancer and lung cancer, more preferably lung cancer, and particularly preferably non-small-cell lung cancer.

The pharmaceutical composition and/or the kit of the invention may be administered orally or parenterally.

When the pharmaceutical composition and/or kit of the invention is used, the given dose of the sulfonamide compound differs depending on the degree of the symptom, age, sex, weight and sensitivity difference of the patient, administration mode, administration period, administration interval, and nature, prescription and type of the pharmaceutical formulation and the type of the active ingredient. Usually, but without limitation, the dose of the sulfonamide compound is 10-6000 mg/day, preferably 50-4000 mg/day, more preferably 50-2000 mg/day for an adult (weight 60 Kg), which may be administered once to three times a day.

When using the pharmaceutical composition and/or the kit of the invention, the given dose of the substance having an EGF inhibitory activity is usually, but not particularly limited to, 10-6000 mg/day, preferably 50-4000 mg/day, more preferably 50-2000 mg/day for an adult, which may be administered once to three times a day.

When using the pharmaceutical composition and/or the kit of the invention, the given dose of the EGFR kinase inhibitor is usually, but not particularly limited to, 10-6000 mg/day, preferably 50-4000 mg/day, more preferably 50-2000 mg/day for an adult, which may be administered once to three times a day.

When using the pharmaceutical composition and/or the kit of the invention, the given dose of the anti-EGFR antibody is usually, but not particularly limited to, 1-6000 mg/day, preferably 10-2000 mg/day, more preferably 10-1000 mg/day for an adult, which may be administered once to three times a day.

The amount of the sulfonamide compound used is not particularly limited, and differs depending on the individual combination with a substance having an EGF inhibitory activity. For example, the amount of the sulfonamide compound is about 0.01-100 times (weight ratio), more preferably about 0.1-10 times (weight ratio) of the amount of the substance having an EGF inhibitory activity.

The pharmaceutical composition of the invention may be made into various dosage forms, for example, into solid oral formulations or parenteral formulations such as injection, suppository, ointment and skin patch.

Furthermore, the sulfonamide compound and the substance having an EGF inhibitory activity contained in the kit of the invention may individually be made into various dosage forms, for example, into solid oral formulations or parenteral formulations such as injection, suppository, ointment and skin patch.

In order to prepare a solid oral formulation, an excipient, and if necessary, a binder, disintegrant, lubricant, colorant, a flavoring agent or the like may be added to a principal agent, and then made into a tablet, a coated tablet, granule, subtle granule, powder, a capsule or the like according to a conventional method. In addition, a non-solid oral formulation such as a syrup agent can also be prepared appropriately.

For example, lactose, cornstarch, sucrose, glucose, sorbit, crystalline cellulose, silicon dioxide or the like may be used as the excipient; for example, polyvinyl alcohol, ethyl cellulose, methyl cellulose, gum arabic, hydroxypropyl cellulose, hydroxypropylmethyl cellulose or the like may be used as the binder; for example, magnesium stearate, talc, silica or the like may be used as the lubricant; those that are allowed to be added to pharmaceutical preparations may be used as the colorant; and for example, cocoa powder, menthol, aromatic acid, peppermint oil, camphor, cinnamon powder or the like may be used as the flavoring agent. Of course, if necessary, these tablets and granule may be coated appropriately with sugar coating, gelatin coating or else.

When an injection is to be prepared, if necessary, the principal agent may be added with a pH adjuster, a buffer, a suspending agent, a solubilizing aid, a stabilizer, an isotonizing agent, a preservative or the like, and may be made into an intravenously, subcutaneously or intramuscularly injection or an intravenous drip injection according to a conventional method. In this case, if necessary, it is also prepared a lyophilized form by a conventional technique.

Examples of the suspending agent may include methyl cellulose, Polysorbate 80, hydroxyethyl cellulose, gum arabic, powdered tragacanth, sodium carboxy methyl cellulose and polyoxyethylene sorbitan monolaurate.

Examples of the solubilizing aid may include polyoxyethylene hydrogenated castor oil, Polysorbate 80, nicotine acid amide, polyoxyethylene sorbitan monolaurate, macrogol, and ethyl ester of castor oil fatty acid.

Examples of the stabilizer may include sodium sulfite and sodium metasulfite; examples of the preservative may include methyl parahydroxybenzoate, ethyl parahydroxybenzoate, sorbic acid, phenol, cresol and chlorocresol.

Besides the sulfonamide compound and the substance having an EGF inhibitory activity, the pharmaceutical composition and/or the kit of the invention can also comprise a packaging container, an instruction, a package insert or

the like. The packaging container, the instruction, the package insert or the like may include description of combinations for combinational use of the compound, and usage and dosage in the case of administering separate substances in combination or in the case of administering them in the form of a mixture. The usage and dosage may be described referring to the related description above.

The kit of the invention may also comprise: (a) at least one selected from the group consisting of a packaging container, an instruction and a package insert describing combinational use of the sulfonamide compound and the substance having an EGF inhibitory activity; and (b) a pharmaceutical composition comprising the sulfonamide compound. The kit is useful for treating cancer. The pharmaceutical composition comprising the sulfonamide compound is useful for treating cancer. The packaging container, the instruction, the package insert or the like may include the description of combinations for combinational use of the sulfonamide compound and the substance having an EGF inhibitory activity, and usage and dosage for combinational use in the case of administering separate substances in combination or in the case of administering them in the form of a mixture. The usage and dosage may be described referring to the related description above.

The present invention also comprises use of the sulfonamide compound for producing a pharmaceutical composition in combination with the substance having an EGF inhibitory activity. According to the use of the invention, the pharmaceutical composition is useful for treating cancer.

The sulfonamide compound and substance having an EGF inhibitory activity can be used in a method for treating cancer comprising simultaneously or separately administering the sulfonamide compound and the substance having an EGF inhibitory activity to a patient. The route and the method for administering the sulfonamide compound and the substance having an EGF inhibitory activity are not particularly limited but reference may be made to the description of the pharmaceutical composition of the invention above.

Hereinafter, the present invention will be described by way of specific examples, although the present invention is not limited thereto.

EXAMPLE 1: Combinational use of E7820 and gefitinib on proliferation of human non-small-cell lung cancer cell line (PC9) *in vitro*

Human non-small-cell lung cancer cell line PC9 (obtained from Immuno-Biological laboratories Co., Ltd.) was suspended in RPMI1640 (containing 10% FBS) to 1 x $10^4$ cells/ml, and 100 μl each of this solution was added to each well of a 96 well plate for cultivation in a 5% carbon dioxide incubator at 37°C. Six hours after the start of the cultivation, the medium was removed. Then, a solution containing E7820, a solution containing gefitinib (Iressa® purchased from AstraZeneca) and a solution containing both compounds, i.e., E7820 and gefitinib, were each diluted in a culture solution (RPMI1640 (containing 10% FBS)). These diluted solutions were added to the cells above for further cultivation.

Three days later, 10 μl of cell counting kit-8 solution (Cell Counting Kit-8, Wako Pure Chemical Industries) was added, cultured for 6 hours at 37°C, and absorbance at 450 nm was determined with a plate reader (Corona Electric Co., Ltd.).

The effect of combinational use was assessed by Isobologram method (Figure 1, Steel GG et al: Int J Radiat Oncol Biol Phys 5: 85-91, 1979., Kano Y, et al: Int J Cancer 50: 604-610, 1992.). According to this method, three types of curves (mode I, mode IIa and mode IIb) were calculated from the curve of growing cell counts with respect to the concentration of each compound, showing theoretical concentrations for inhibiting 50% cell growth by combinational use. Therefore, when a plot of 50% inhibitory concentration (IC50) in the well where the compounds are used in combination is within the region surrounded by these three lines (additive region) (Figure 1, "Pb"), it is considered to have an additive effect. When a plot of 50% inhibitory concentration (IC50) in a well where the compounds are used in combination is within a region innermost to the mode curves (Figure 1, "Pa"), it is considered to have a synergistic effect. When a plot of 50% inhibitory concentration (IC50) in a well where the compounds are used in combination is within a region outside the mode curves (Figure 1, "Pc"), it is considered to have an antagonistic effect. When a plot of 50% inhibitory concentration (IC50) in a well where the compounds are used in combination exceeds the IC50 of each compound, it is considered to have a protection effect (Figure 1, "Pd").

As a result, E7820 was found to have a synergistic effect upon combinational use with gefitinib (Figure 2, "Combi.").

EXAMPLE 2: Combinational use of E7070 and gefitinib on *in vitro* proliferarion of human non-small-cell lung cancer cell line (PC9)

Human non-small-cell lung cancer cell line PC9 was suspended in RPMI1640 (containing 10% FBS) to 1 x $10^4$ cells/ml, and 100 μl each of this solution was added to each well of a 96 well plate for cultivation in a 5% carbon dioxide incubator at 37°C. Twenty-four hours after the start of the cultivation, the medium was removed. A solution containing E7070, a solution containing gefitinib (purchased from AstraZeneca) and a solution containing both compounds, i.e.,

E7070 and gefitinib, were each diluted in a culture solution (RPMI1640 (containing 10% FBS)). Then, these diluted solutions were added to the cells above for further cultivation.

Three days later, the cells were washed with 100μl PBS/well, and immobilized with 10% trichloroacetic acid. Then, the cells were stained by SRB technique to determine absorbance at 550 nm with a plate reader.

The effect of combinational use was assessed by Isobologram method.

As a result, E7070 was found to have a synergistic effect upon combinational use with gefitinib (Figure 3, "Combi.")).

EXAMPLE 3: Combinational use of E7070 and erlotinib on *in vitro* proliferarion of human non-small-cell lung cancer cell line (PC9)

Human non-small-cell lung cancer cell line PC9 was suspended in RPMI1640 (containing 10% FBS) to 1 x $10^4$ cells/ml, and 100 μl each of this solution was added to each well of a 96 well plate for cultivation in a 5% carbon dioxide incubator at 37°C. Twenty-four hours after the start of the cultivation, the medium was removed. A solution containing E7070, a solution containing erlotinib (Tarceva® purchased from Genentech) and a solution containing both compounds, i.e., E7070 and erlotinib, were each diluted in a culture solution (RPMI1640 (containing 10% FBS)). Then, these diluted solutions were added to the cells above for further cultivation.

Three days later, the cells were washed with 100 μl PBS/well, and immobilized with 10% trichloroacetic acid. Then, the cells were stained by SRB technique to determine absorbance at 550 nm with a plate reader.

The effect of combinational use was assessed by Isobologram method.

As a result, E7070 was found to have a synergistic effect upon combinational use with erlotinib (Figure 4, "Combi.")).

EXAMPLE 4: Combinational use of E7820 and gefitinib in subcutaneous transplant model *(in vivo)* of human non-small-cell lung cancer cell line (PC9)

Human non-small-cell lung cancer cell line PC9 (obtained from Immuno-Biological laboratories Co., Ltd.) was cultured in RPMI1640 (containing 10% FBS) in a 5% carbon dioxide incubator at 37°C to about 80% confluence, and the cells were collected with trypsin-EDTA. Using a phosphate buffer, 5 x $10^7$ cells/mL suspension was prepared, and 0.1 mL each of the resulting cell suspension was subcutaneously transplanted to a nude mouse at the side of its body. Eight days after the transplantation, E7820 and gefitinib were orally administered alone or in combination for 50 mg/kg twice a day for 2 weeks and for 75 mg/kg once a day for 2 weeks, respectively. The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{Minor axis of tumor})^2 (\text{mm}^2)/2$$

$$\text{Relative Tumor Volume RTV} = \text{Tumor volume on measurement day/Tumor volume on the first administration day}$$

If statistically significant interaction was observed in the combinational use group by two-way ANOVA, a synergistic effect was considered to exist between E7820 and gefitinib.

As a result, E7820 was found to produce a synergistic effect when used in combination with gefitinib, and their combinational use showed a superior anti-tumor effect as compared with the effect obtained with E7820 or gefitinib alone (Table 5 and Figure 5). In addition, combinational use of E7820 and gefitinib also showed a remarkable anti-tumor effect that cannot be seen with gefitinib alone (Table 5 and Figure 5).

Table 5

| Administered compound | Relative tumor volume on Day 15 Average ± standard deviation | Two-way ANOVA |
|---|---|---|
| Control (untreated) | 4.12 ± 0.68 | |
| E7820 50 mg/kg | 2.57 ± 0.41 | |
| Gefitinib 75 mg/kg | 0.36 ± 0.12 | |
| E7820 50 mg/kg + gefitinib 75 mg/kg | 12 ± 0.03 | p < 0.01 Synergistic effect |

Table 5 shows anti-tumor effects obtained by the use of E7820 alone, the use of gefitinib alone and the combinational use of E7820 and gefitinib in subcutaneous transplant models of human non-small-cell lung cancer cell line (PC9). The first day of administration was considered Day 1.

From the obtained results, the combination of E7820 and gefitinib provides a pharmaceutical composition and a kit that show a remarkable anti-tumor activity, and a method for treating cancer, and thus the pharmaceutical composition, the kit and the method of the invention can be used for treating cancer.

EXAMPLE 5: Combinational use of E7820 and gefitinib in subcutaneous transplant model *(in vivo)* of human non-small-cell lung cancer cell line (A549)

Human non-small-cell lung cancer cell line A549 (purchased from Dainippon Pharmaceutical) was cultured in RPMI1640 (containing 10% FBS) in a 5% carbon dioxide incubator at 37°C to about 80% confluence, and the cells were collected with trypsin-EDTA. Using a phosphate buffer containing 50% matrigel, 5 x $10^7$ cells/mL suspension was prepared, and 0.1 mL each of the resulting cell suspension was subcutaneously transplanted to a nude mouse at the side of its body. Ten days after the transplantation, E7820 and gefitinib were orally administered alone or in combination for 50 mg/kg twice a day for 3 weeks and for 75 mg/kg once a day for 3 weeks, respectively. The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{minor axis of tumor})^2 (\text{mm}^2)/2$$

$$\text{Relative Tumor Volume RTV} = \text{Tumor volume on measurement day/Tumor volume on the first administration day}$$

If statistically significant interaction was observed in the combinational use group by two-way ANOVA, a synergistic effect is considered to exist between E7820 and gefitinib.

As a result, E7820 was found to produce a synergistic effect when used in combination with gefitinib, and their combinational use showed a superior anti-tumor effect as compared with the effect obtained with E7820 or gefitinib alone (Table 6 and Figure 6). In addition, combinational use of E7820 and gefitinib also showed a remarkable anti-tumor effect that cannot be seen with gefitinib alone (Table 6 and Figure 6).

Table 6

| Administered compound | Relative tumor volume on Day 22 Average ± standard deviation | Two-way ANOVA |
|---|---|---|
| Control (untreated) | 4.95 ± 0.86 | |
| E7820 50 mg/kg | 3.69 ± 0.68 | |
| Gefitinib 75 mg/kg | 2.26 ± 0.59 | |
| E7820 50 mg/kg + gefitinib 75 mg/kg | 1.05 ± 0.21 | p < 0.01 Synergistic effect |

Table 6 shows anti-tumor effects obtained by the use of E7820 alone, the use of gefitinib alone and the combinational use of E7820 and gefitinib in subcutaneous transplant models of human non-small-cell lung cancer cell line (A549). The first day of administration was considered Day 1.

From the obtained results, the combination of E7820 and gefitinib provides a pharmaceutical composition and a kit that show a remarkable anti-tumor activity, and a method for treating cancer, and thus the pharmaceutical composition, the kit and the method of the invention can be used for treating cancer.

EXAMPLE 6: Combinational use of E7820 and erlotinib in subcutaneous transplant model *(in vivo)* of human non-small-cell lung cancer cell line (A549)

Human non-small-cell lung cancer cell line A549 (purchased from Dainippon Pharmaceutical) was cultured in RPMI1640 (containing 10% FBS) in a 5% carbon dioxide incubator at 37°C to about 80% confluence, and the cells were collected with trypsin-EDTA. Using a phosphate buffer containing 50% matrigel, 5 x $10^7$ cells/mL suspension was prepared, and 0.1 mL each of the resulting cell suspension was subcutaneously transplanted to a nude mouse at the side of its body. Seventeen days after the transplantation, E7820 and erlotinib were orally administered alone or in combination for 50 mg/kg twice a day for 2 weeks and for 100 mg/kg once a day for 2 weeks, respectively. The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{Minor axis of tumor})^2 (\text{mm}^2)/2$$

$$\text{Relative Tumor Volume RTV} = \text{Tumor volume on measurement day/Tumor volume on the first administration day}$$

If statistically significant interaction was observed in the combinational use group by two-way ANOVA, a synergistic effect was considered to exist between E7820 and erlotinib.

As a result, E7820 was found to produce a synergistic effect when used in combination with erlotinib and their combinational use showed a superior anti-tumor effect as compared with the effect obtained with E7820 or erlotinib alone (Table 7 and Figure 7). In addition, combinational use of E7820 and erlotinib also showed a remarkable anti-tumor effect that cannot be seen with erlotinib alone (Table 7 and Figure 7).

Table 7

| Administered compound | Relative tumor volume on Day 15 Average ± standard deviation | Two-way ANOVA |
|---|---|---|
| Control (untreated) | 3.48 ± 0.61 | |
| E7820 50 mg/kg | 2.62 ± 0.07 | |
| Erlotinib 100 mg/kg | 1.94 ± 0.19 | |
| E7820 50 mg/kg + erlotinib 100 mg/kg | 0.91 ± 0.09 | P < 0.01 Synergistic effect |

Table 7 shows anti-tumor effects obtained by the use of E7820 alone, the use of erlotinib alone and the combinational use of E7820 and erlotinib in subcutaneous transplant models of human non-small-cell lung cancer cell line (A549). The first day of administration was considered Day 1.

From the obtained results, the combination of E7820 and erlotinib provides a pharmaceutical composition and a kit that show a remarkable anti-tumor activity, and a method for treating cancer, and thus the pharmaceutical composition, the kit and the method of the invention can be used for treating cancer.

EXAMPLE 7: DNA microarray analysis

(1) Cell culture, compound treatment and RNA extraction

For the purpose of examining changes in the gene expression induced by the compounds by a DNA microarray analysis, human colon cancer-derived cell line HCT116 (American Type Culture Collection, Manassas, VA, U.S.A.) and human leukemia-derived cell line MOLT-4 (American Type Culture Collection, Manassas, VA, U.S.A.) were cultured in RPMI-1640 media supplemented with 10% fetal bovine serum, 100 units/ml penicillin and 100 μg/ml streptomycin. The following cultivation and compound treatment took place in an incubator set to 5% $CO_2$ and 37°C. The HCT116 cells and the MOLT-4 cells were seeded on 10 cm-diameter cell culture dishes at 2.0 x $10^6$ cells/dish, cultured for 24 hours and subjected to the following compound treatments.

For the HCT116 cells, 12 compounds, i.e., E7820 (0.8 μM), E7070 (0.8 μM), LY295501 (30 μM), CQS (8 μM), adriamycin (0.2 μM), daunomycin (0.2 μM), ICRF154 (80 μM), ICRF159 (80 μM), kenpaullone (10 μM), alsterpullone (10 μM), trichostatin A (0.1 μM) and rapamycin (80 μM) were assessed. On the other hand, for the MOLT-4 cells, E7070 (0.8 μM) was assessed. Herein, adriamycin and daunomycin are compounds known as DNA intercalative DNA topoisomerase II inhibitors, ICRF154 and ICRF 159 are compounds known as catalytic DNA topoisomerase II inhibitors, kenpaullone and alsterpullone are compounds known as cyclin-dependent kinase (CDK) inhibitors, trichostatin A is a compound known as a histone deacetylase inhibitor and rapamycin is a compound known as an mTOR/FRAP inhibitor. The concentration of each compound used for the treatment was set to three to five-fold the 50% growth inhibitory concentration of each compound to the HCT116 cells (based on three days of antiproliferative activity using WST-8). The cells were collected 24 hours after the treatment at the concentration indicated in parentheses following each compound name above. Similarly, cells cultured for 24 hours without the addition of any compound were also collected.

Extraction of total RNA from the collected cells was performed using TRIZOL reagent (Invitrogen) according to the attached instruction.

(2) Analysis of gene expression using DNA microarray

The resulting RNA was dissolved in 100 μl of diethylpyrocarbonate (DEPC)-treated sterilized water, purified using an RNeasy column (QIAGEN), and double-stranded cDNA was synthesized using SuperScript Choice System (Invitrogen) and T7-d$(T)_{24}$ primers.

First, to 10 μg RNA, 5 μM T7-d$(T)_{24}$ primer, 1x First strand buffer, 10 mM DTT, 500 μM dNTP mix and 20 units/μl SuperScript II Reverse Transcriptase were added and reacted at 42°C for an hour to synthesize single-stranded DNA. Subsequently, Ix Second strand buffer, 200 μM dNTP mix, 67 U/ml DNA ligase, 270 U/ml DNA polymerase I and 13 U/ml RNase H were added and reacted at 16°C for two hours to synthesize double-stranded cDNA. Furthermore, 67 U/ml T4 DNA polymerase I was added, reacted at 16°C for 5 minutes and then 10 μl of 0.5 M EDTA was added to terminate the reaction.

The obtained cDNA was purified with phenol/chloroform, and subjected to labeling reaction with biotinylated UTP and CTP using RNA Transcript Labeling Kit (Enzo Diagnostics) according to the attached instruction. The reaction product was purified using an RNeasy column, heated in 200 mM Tris acetic acid (pHB.1), 150 mM magnesium acetate and 50 mM potassium acetate at 94°C for 35 minutes for fragmentation of the cRNA.

The fragmented cRNA was hybridized to GeneChip (Affymetrix) Human Focus array in 100 mM MES, 1 M sodium salt, 20 mM EDTA and 0.01% Tween 20 at 45°C for 16 hours. After the hybridization, GeneChip was washed and stained according to protocol Midi_euk2 attached to the Affymetrix fluidics station. For staining, streptavidin-phycoerythrin and biotinylated anti-streptavidin goat antibody were used. The stained GeneChip was scanned using HP confocal microscope with argon ion laser (Hewlett Packard) to determine fluorescence intensity. Measurement took place at excitation and emission wavelengths of 488 nm and 570 nm, respectively.

All of the quantitative data analyses were carried out using GeneChip software (Affymetrix) and Gene Spring (Silicongenetics). GeneChip software was used for assessing changes in the gene expression induced by each compound, where gene expression was judged to have significantly "increased" or "decreased" when the quantified values in the two conditions, i.e., between the compound-treated group and the untreated group, were twice or more as different. Gene Spring was used for assessing the similarity of changes in gene expression induced by each compound, where hierarchical cluster analysis was conducted based on changes in the expressions of all genes on the Human Focus Array.

The results from the hierarchical cluster analysis for the HCT116 cells are shown in Figure 8.

As a result of the analysis, adriamycin and daunomycin, ICRF154 and ICRF159, and Kenpaullone and alsterpullone, each pair having the same action mechanism, gave similar genetic alterations (Figure 8). Thus, compounds having the same action mechanism were confirmed to give similar genetic alterations.

E7070, E7820, LY295501 and CQS gave similar genetic alterations (Figure 8). Therefore, E7070, E7820, LY295501 and CQS were considered to have the same or similar action mechanisms according to this analysis, strongly

suggesting that they give the same or similar genetic alterations and effects.

EXAMPLE 8: DNA microarray analysis

HCT116 cells were cultured in an RPMI-1640 medium supplemented with 10% fetal bovine serum, 100 units/ml penicillin and 100 μg/ml streptomycin. The following cultivation and compound treatment were carried out in an incubator at 5% $CO_2$ and 37°C. HCT116 cells were seeded in 10 cm-diameter cell culture dishes at 2.0 x $10^6$ cells/dish, cultured for 24 hours and subjected to the following compound treatment.

In this example, changes in the gene expression of HCT116 cells upon treatments with 12 compounds, i.e., E7820 (0.16 μM), E7070 (0.26 μM), LY186641 (59 μM), LY295501 (24 μM), LY-573636 (9.6 μM), CQS (4.0 μM), MST16 (100 μM), etoposide (3.6 μM), ethoxzolamide (410 μM), capsaicin (280 μM), trichostatin A (0.16 μM) and kenpaullone (7.1 μM) were examined.

MST16 is a compound known as a catalytic DNA topoisomerase II inhibitor, etoposide is a compound known as a DNA topoisomerase II inhibitor that induces formation of a cleavable complex, ethoxzolamide is a compound known as a carbonic anhydrase inhibitor, capsaicin is a compound known as a tumor-specific plasma membrane NADH oxidase inhibitor, trichostatin A is a compound known as a histone deacetylase inhibitor and kenpaullone is a compound known as a cyclin-dependent kinase (CDK) inhibitor.

The concentration of each compound used for the treatment was set to twice the 50% growth inhibitory concentration of each compound to the HCT116 cells (based on three days of antiproliferative activity using MTT). The cells were collected 24 hours after the treatment at the concentration indicated in parentheses following each compound name above. Similarly, cells cultured for 24 hours without the addition of any compound were also collected.

Total RNA extraction from the collected cells was performed using TRIZOL reagent (Invitrogen) according to the attached instruction.

Gene expression analysis using a DNA microarray was carried out in the same manner as "(2) Analysis of gene expression using DNA microarray" in Example 7.

This example was conducted for each sample in duplicate (for the convenience of the experiment, samples were given branch numbers like control-1, control-2, E7070-1, E7070-2 and so on for distinction). Then, GeneChip (Affymetrix) system (Human Focus array) was used for analyzing changes in the gene expression induced by each compound.

Twenty-six ".cel" files obtained in this example (13 samples (a control + 12 compounds) x 2) were subjected to RMA method (robust multi-array average method (Biostatistics (2003), 4, 249-264)) for normal distribution at probe level, and then the logarithm value of the signal intensity at gene level was calculated. Next, the logarithm value of the signal intensity of the untreated group (control-1) was subtracted from the logarithm value of the signal intensity of the compound-treated group for each gene to obtain the logarithm value of the signal ratio of the compound-treated group to control-1. Then, cosine correlation coefficients were calculated as correlation coefficients between the experiments (Figure 9). Based on these correlation coefficients, hierarchical cluster analysis was performed according to UPGMA method (Unweighted Pair Group Method with Arithmetic mean method) (Figure 10). Control-2 was also subjected to similar calculation (Figures 11 and 12). The softwares used were R 2.0.1 (http://www.r-project.org/) and affy package 1.5.8 (http://www.bioconductor.org).

In Figures 9-12, "LY1" represents LY186641, "LY2" represents LY295501, "LY5" represents LY573636, "CAI" represents ethoxzolamide, "Cap" represents capsaicin, "MST" represents MST16, "Etop" represents etoposide, "TSA" represents trichostatin A, and "Kenp" represents kenpaullone. In Figures 10 and 12, "de hclust (*, "average")" is a command upon statistical analysis, showing that clustering analysis is conducted by R using the average value of the duplicate experiment data.

As a result of the analysis, E7070, E7820, LY186641, LY295501, LY573636 and CQS showed very similar genetic alterations for the HCT116 cells, and were found to be different from the profiles of any of the other compounds (MST16, etoposide, ethoxzolamide, capsaicin, trichostatin A and kenpaullone) (Figures 9-12). Thus, by this analysis, E7070, E7820, LY186641, LY295501, LY573636 and CQS were considered to have the same or similar action mechanisms, strongly suggesting that they give the same or similar genetic alterations and effects.

EXAMPLE 9: Experiment on cancer cell line panels

Human cancer cell panels from 36 cell lines were used to examine correlation of antiproliferative activities among E7820, E7070, CQS, LY186641 and LY295501.
The 36 types of cancer cell lines used were DLD-1, HCT15, HCT116, HT29, SW480, SW620 and WiDr (which are human colon cancer cell lines), A427, A549, LX-1, NCI-H460, NCI-H522, PC-9 and PC-10 (which are human lung cancer cell lines), GT3TKB, HGC27, MKN1, MKN7, MKN28 and MKN74 (which are human gastric cancer cell lines), AsPC-1, KP-1, KP-4, MiaPaCaII, PANC-1 and SUIT-2 (which are human pancreas cancer cell lines), BSY-1, HBC5, MCF-7, MDA-MB-231, MDA-MB-435 and MDA-MB-468 (which are human breast cancer cell lines), and CCRF-CEM, HL60, K562 and MOLT-4 (which are human leukemia cell lines). All of the cells were cultured using RPMI-1640 media supplemented with 10% fetal bovine serum, 100 units/ml penicillin and 100 μg/ml streptomycin under the conditions of 5% $CO_2$ and 37°C (Table 8).

Table 8

**36 human cancer cell lines tested in 3-day MTT assays**

| **Colon** | **Stomach** | **Breast** |
|---|---|---|
| DLD-1 (1250/well, 16.8 h) | GT3TKB (2000/well, 21.1 h) | BSY-1 (2000/well, 46.1 h) - |
| HCT15 (1500/well, 14.5 h) | HGC27 (1500/well, 14.6 h) | HBC5 (2000/well, 31.8 h) |
| HCT116 (1250/well, 13.4 h) | MKN1 (4000/well, 35.9 h) | MCF-7 (3000/well, 29.5 h) |
| HT29 (2500/well, 19.8 h) | MKN7 (3000/well, 37.4 h) | MDA-MB231 (2000/well, 21.6 h) |
| SW480 (3000/well, 19.5 h) | MKN28 (2000/well, 22.7 h) | MDA-MB-435 (3000/well, 24.4 h) |
| SW620 (2500/well, 17.3 h) | MKN74 (4000/well, 24.8 h) | MDA-MB-468 (3000/well, 34.2 h) |
| WiDr (2000/well, 18.9 h) | | |
| **Lung** | **Pancreas** | **Leukemia** |
| A427 (2500/well, 32.4 h) | AsPC-1 (2500/well, 28.4 h) | CCRF-CEM (1500/well, 27.2 h) |
| A549 (1250/well, 18.9 h) | KP-1 (2000/well, 24.8 h) | HL60 (1500/well, 29.5 h) |
| LX-1 (2000/well, 17.2 h) | KP-4 (2000/well, 16.7 h) | K562 (1500/well, 20.6 h) |
| NCI-H460 (1000/well, 13.6 h) | MiaPaCall (2500/well, 19.1 h) | MOLT-4 (1500/well, 22.3 h) |
| NCI-H522 (4000/well, 80.4 h) | PANC-1 (2500/well, 27.9 h) | |
| PC-9 (2000/well, 23.7 h) | SUIT-2 (2000/well, 15.6 h) | |
| PC-10 (2000/well, 24.0 h) | | |

**Cell line (initial cell number, doubling time)**

Table 8 shows the types, seeded cell numbers and doubling times of the human cancer cell lines in the human cancer cell line panels.

The cells were seeded on a 96-well microplate (flat bottom) at the number indicated in Table 8 (50 μl/well). Twenty-four hours later, they were added with a 3-fold dilution series of each compound (50 μl/well). Seventy-two hours later, WST-8 (10 μl/well) was added and absorbance at 450 nm was determined. The 50% growth inhibitory concentrations to all of the 36 cancer cell lines were obtained by a least square method and their patterns were compared between the compounds. As the correlation index, Pearson's correlation coefficients were used (Paull, K. D. et al. Display and analysis of patterns of differential activity of drugs against human tumor cell lines: development of mean graph and COMPARE algorithm. J. Natl. Cancer Inst. 1989, 81, 1088-1092; Monks, A. et al. Feasibility of a high-flux anticancer drug screen using a diverse panel of cultured human tumor cell lines. J. Natl. Cancer Inst. 1991, 83, 757-766).

As a result, E7070, E7820, LY186641, LY295501 and CQS showed high correlation coefficients in antiproliferative activities against each cancer cell line (Table 9). Thus, by this analysis, E7070, E7820, LY186641, LY295501 and CQS were considered to have the same or similar action mechanisms, strongly suggesting that they give the same or similar genetic alterations and effects.

Table 9

| | F7070 | E7870 | COS | LY186641 | LY295501 |
|---|---|---|---|---|---|
| E7070 | 1.00 | 0.98 | 0.97 | 0.93 | 0.80 |
| E7820 | 0.98 | 1.00 | 0.96 | 0.95 | 0.82 |
| CQS | 0.97 | 0.96 | 1.00 | 0.92 | 0.82 |
| LY 186641 | 0.93 | 0.95 | 0.92 | 1.00 | 0.81 |
| LY795501 | 0.80 | 0.82 | 0.82 | 0.81 | 1.00 |

Table 9 shows correlation coefficients between the compounds (E7070, E7820, CQS, LY 186641 and LY295501) on the human cancer cell line panels.

EXAMPLE 10: Cross-resistance in E7070-resistant cell line

An E7070-resistant cell line was used to assess the antiproliferative activities of E7820, LY186641, LY295501, LY-ASAP and CQS. HCT116-C9 was a substrain separated from human colon cancer-derived HCT116 (American Type Culture Collection, Manassas, VA, U.S.A.). This HCT116-C9 was cultured in the presence of E7070 while increasing the E7070 concentration by degrees, thereby obtaining E7070-resistant substrains HCT116-C9-C1 and HCT116-C9-C4 (Molecular Cancer Therapeutics, 2002, 1, 275-286).

Three cell lines, i.e., HCT116-C9, HCT116-C9-C1 and HCT116-C9-C4, were each seeded at 3000 cells/well onto a 96-well microplate (flat bottom) (50μl/well). Twenty-four hours later, they were added with a 3-fold dilution series of each compound (50 μl/well). Seventy-two hours later, the antiproliferative activities were assessed by MTT method (Mossmann T., J. Immunol. Methods, 1983, 65, 55-63). The 50% growth inhibitory concentrations to the cancer cells were obtained by a least square method.

As a result, the antiproliferative activity, i.e., IC50, of E7070 to HCT116-C9 (C9) was 0.127 μM. On the other hand, activities to HCT116-C9-C1 (C9C1) and HCT116-C9-C4 (C9C4) were IC50 = 31.9 μM and 26.9 μM, respectively, confirming that the antiproliferative activities of E7070 to C9C1 and C9C4 were remarkably low (Figure 13). The antiproliferative activities of E7820, CQS, LY186641, LY295501 and LY-ASAP to HCT116-C9 were IC50 = 0.080 μM, 1.73 μM, 33.6 μM, 10.9 μM and 1.63 μM, respectively while their activities to HCT116-C9-C1 were IC50 = 51.2 μM, 634 μM, 134 μM, 111 μM and 113 μM, respectively and their activities to HCT116-C9-C4 were IC50 = 52.8 μM, 517 μM, 138 μM, 110 μM and 90.3 μM, respectively. Therefore, the antiproliferative activities of E7820, CQS, LY186641, LY295501 and LY-ASAP to C9C1 and C9C4 were far lower than those to C9 (Figure 13). Thus, E7070, E7820, LY186641, LY295501, LY-ASAP and CQS were considered to have the same or similar action mechanisms, strongly suggesting that they give the same or similar genetic alterations and effects.

EXAMPLE 11: Cross-resistance in E7070-resistant cell line

In exactly the same manner as in Example 10, an E7070-resistant cell line was used to assess the antiproliferative activities of LY573636 as well as those of E7070.

As a result, the antiproliferative activities of E7070 to HCT116-C9-C1 and HCT116-C9-C4 (IC50 = 32.7 μM and 28.0 μM, respectively) were again confirmed to be remarkably lower than the activity to HCT116-C9 (IC50 = 0.127 μM) (Figure 14). The antiproliferative activities of LY573636 to HCT116-C9-C1 and HCT116-C9-C4 (IC50 = 264 μM and 240 μM, respectively) were also remarkably lower than the activity to HCT116-C9 (IC50 = 5.11 μM) (Figure 14). Thus, LY573636 was considered to have the same or similar action mechanism as that of E7070, strongly suggesting that it gives the same or similar genetic alteration and effect.

These results (Examples 7-11) confirmed that E7070, E7820, LY186641, LY295501, LY-ASAP, LY573636, CQS or a combination thereof give the same or similar genetic alterations and thus the same or similar actions and effects.

Accordingly, similar to E7820 and E7070 (Examples 1-6, 12 and 13), a sulfonamide compound, preferably E7820, E7070, LY186641, LY295501, LY-ASAP, LY573636, CQS or a combination thereof was found to show a remarkable anti-tumor activity upon combinational use with a substance having an EGF inhibitory activity, preferably gefitinib, erlotinib or cetuximab.

EXAMPLE 12: Combinational use of E7820 and cetuximab in subcutaneous transplant model *(in vivo)* of human colon cancer cell line (WiDr)

Human colon cancer cell line WiDr (obtained from Dainippon Pharmaceutical) was cultured in RPMI1640 (containing 10% FBS) in a 5% carbon dioxide incubator at 37°C to about 80% confluence, and the cells were collected with trypsin-EDTA. Using a phosphate buffer, $5 \times 10^7$ cells/mL suspension was prepared, and 0.1 mL each of the resulting cell suspension was subcutaneously transplanted to a nude mouse at the side of its body. Ten days after the transplantation, E7820 and cetuximab (Erbitux® purchased from Merck) were administered alone or in combination. E7820 was orally administered at 50 mg/kg twice a day for 2 weeks while cetuximab was intraperitoneally administered at 100 mg/kg twice a week for 2 weeks.

The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{Minor axis of tumor})^2 (\text{mm}^2)/2$$

$$\text{Relative Tumor Volume RTV} = \text{Tumor volume on measurement day/Tumor volume on the first administration day}$$

When statistically significant interaction was observed in the combinational use group by two-way ANOVA, a synergistic effect was considered to exist.

As a result, combinational use of E7820 and cetuximab showed a superior anti-tumor effect as compared with the effect obtained with E7820 or cetuximab alone (Table 10).

Table 10

| Administered drug | Relative tumor volume on Day 15 Average ± standard deviation |
|---|---|
| Control (untreated) | 4.1 ± 0.7 |
| E7820 50 mg/kg | 3.3 ± 0.6 |
| Cetuximab 100 mg/kg | 3.2 ± 0.4 |
| E7820 50 mg/kg + cetuximab 100 mg/kg | 2.7 ± 0.4 |

Table 10 shows anti-tumor effects obtained by the use of E7820 alone, the use of cetuximab alone and the combinational use of E7820 and cetuximab in subcutaneous transplant models of human colon cancer cell line (WiDr). The first day of administration was considered Day 1.

EXAMPLE 13: Combinational use of E7820 and cetuximab in subcutaneous transplant model *(in vivo)* of human renal cancer cell line (ACHN)

Human renal cancer cell line ACHN (obtained from Dainippon Pharmaceutical) was cultured in RPMI1640 (containing 10% FBS) in a 5% carbon dioxide incubator at 37°C to about 80% confluence, and the cells were collected with trypsin-EDTA. Using a phosphate buffer, 1 x $10^8$ cells/mL suspension was prepared, and 0.1 mL each of the resulting cell suspension was subcutaneously transplanted to a nude mouse at the side of its body. Eight days after the transplantation, E7820 and cetuximab were administered alone or in combination. E7820 was orally administered at 50 mg/kg twice a day for 2 weeks while cetuximab was intraperitoneally administered at 100 mg/kg twice a week for 2 weeks.

The major and minor axes of tumors were measured with Digimatic caliper (Mitsutoyo), and tumor volumes and relative tumor volumes were calculated according to the following formulae.

$$\text{Tumor Volume TV} = \text{Major axis of tumor(mm)} \times (\text{Minor axis of tumor})^2 (\text{mm}^2)/2$$

$$\text{Relative Tumor Volume RTV} = \text{Tumor volume on measurement day/Tumor volume on the first administration day}$$

When statistically significant interaction was observed in the combinational use group by two-way ANOVA, a synergistic effect was considered to exist.

As a result, combinational use of E7820 and cetuximab showed a superior anti-tumor effect as compared with the effect obtained with E7820 or cetuximab alone (Table 11).

Table 11

| Administered drug | Relative tumor volume on Day 15<br>Average ± standard deviation |
|---|---|
| Control (untreated) | 2.0 ± 0.2 |
| E7820 50 mg/kg | 1.3 ± 0.2 |
| Cetuximab 100 mg/kg | 1.4 ± 0.1 |
| E7820 50 mg/kg + cetuximab 100 mg/kg | 0.9 ± 01 |

Table 11 shows anti-tumor effects obtained by the use of E7820 alone, the use of cetuximab alone and the combinational use of E7820 and cetuximab in subcutaneous transplant models of human renal cancer cell line (ACHN). The first day of administration was considered Day 1.

From the obtained results, the combination of E7820 and cetuximab was confirmed to provide a pharmaceutical composition and a kit that show a remarkable anti-tumor activity and a method for treating cancer, and thus the pharmaceutical composition, the kit and the method of the invention can be used for treating cancer.

INDUSTRIAL APPLICABILITY

The present invention provides a pharmaceutical composition and a kit that show a remarkable anti-tumor activity

The pharmaceutical composition and the kit are useful for treating cancer.

## Claims

1. A pharmaceutical composition comprising a sulfonamide compound in combination with a substance having an EGF inhibitory activity,
wherein the sulfonamide compound is:

    N-(3-chloro-1H-indole-7-yl)-4-sulfamoylbenzenesulfonamide, N-(3-cyano-4-methyl-1H-indole-7-yl)-3-cyanobenzenesulfonamide, or a pharmacologically acceptable salt thereof or a solvate thereof,

    and wherein the substance having an EGF inhibitory activity is gefitinib, erlotinib or cetuximab.

2. The pharmaceutical composition according to Claim 1, wherein the pharmaceutical composition is used for treating cancer.

3. A kit comprising a set of a formulation comprising a sulfonamide compound as defined in Claim 1 and a formulation comprising a substance having an EGF inhibitory activity as defined in Claim 1.

4. The kit according to claim 4, for use in treating cancer.

5. Use of a sulfonamide compound as defined in Claim 1 for producing a pharmaceutical composition for use in combination with a substance having an EGF inhibitory activity as defined in Claim 1 in the treatment of cancer.

6. Use of a substance having an EGF inhibitory activity as defined in Claim 1 for producing a pharmaceutical composition for use in combination with a sulfonamide compound as defined in Claim 1 in the treatment of cancer.

7. A sulfonamide compound as defined in Claim 1 for use in combination with a substance having an EGF inhibitory activity as defined in Claim 1 in the treatment of cancer.

8. A substance having an EGF inhibitory activity as defined in Claim 1 for use in combination with a sulfonamide compound as defined in Claim 1 in the treatment of cancer.

9. A pharmaceutical composition comprising a sulfonamide compound as defined in Claim 1 for use in combination with a substance having an EGF inhibitory activity as defined in Claim 1 in the treatment of cancer.

**10.** A pharmaceutical composition comprising a substance having an EGF inhibitory activity as defined in Claim 1 for use in combination with a sulfonamide compound as defined in claim 1 in the treatment of cancer.

## Patentansprüche

**1.** Pharmazeutische Zusammensetzung, die eine Sulfonamid-Verbindung in Kombination mit einer Substanz, die EGF-hemmende Aktivität hat, umfasst,
wobei die Sulfonamid-Verbindung N-(3-Chlor-1H-indol-7-yl)-4-sulfamoylbenzolsulfonamid, N-(3-Cyano-4-methyl-1H-indol-7-yl)-3-cyanobenzolsulfonamid oder ein pharmakologisch verträgliches Salz davon oder ein Solvat davon ist,
und wobei die Substanz, die EGF-hemmende Aktivität hat, Gefitinib, Erlotinib oder Cetuximab ist.

**2.** Pharmazeutische Zusammensetzung gemäß Anspruch 1, wobei die pharmazeutische Zusammensetzung zur Behandlung von Krebs verwendet wird.

**3.** Kit, umfassend ein Set aus einer Formulierung, die eine Sulfonamid-Verbindung, wie sie in Anspruch 1 definiert ist, umfasst, und einer Formulierung, die eine Substanz, die eine EGF-hemmende Aktivität hat, wie sie in Anspruch 1 definiert ist, umfasst.

**4.** Kit gemäß Anspruch 4 zur Verwendung bei der Behandlung von Krebs.

**5.** Verwendung einer Sulfonamid-Verbindung, wie sie in Anspruch 1 definiert ist, zur Herstellung einer pharmazeutischen Zusammensetzung zur Verwendung in Kombination mit einer Substanz, die EGF-hemmende Aktivität hat, wie sie in Anspruch 1 definiert ist, bei der Behandlung von Krebs.

**6.** Verwendung einer Substanz, die EGF-hemmende Aktivität hat, wie sie in Anspruch 1 definiert ist, zur Herstellung einer pharmazeutischen Zusammensetzung zur Verwendung in Kombination mit einer Sulfonamid-Verbindung, wie sie in Anspruch 1 definiert ist, bei der Behandlung von Krebs.

**7.** Sulfonamid-Verbindung, wie sie in Anspruch 1 definiert ist, zur Verwendung in Kombination mit einer Substanz, die EGF-hemmende Aktivität hat, wie sie in Anspruch 1 definiert ist, bei der Behandlung von Krebs.

**8.** Substanz, die EGF-hemmende Aktivität hat, wie sie in Anspruch 1 definiert ist, zur Verwendung in Kombination mit einer Sulfonamid-Verbindung, wie sie in Anspruch 1 definiert ist, bei der Behandlung von Krebs.

**9.** Pharmazeutische Zusammensetzung, die eine Sulfonamid-Verbindung, wie sie in Anspruch 1 definiert ist, umfasst, zur Verwendung in Kombination mit einer Substanz, die EGF-hemmende Aktivität hat, wie sie in Anspruch 1 definiert ist, bei der Behandlung von Krebs.

**10.** Pharmazeutische Zusammensetzung, die eine Substanz, die EGF-hemmende Aktivität hat, wie sie in Anspruch 1 definiert ist, umfasst, zur Verwendung in Kombination mit einer Sulfonamid-Verbindung, wie sie in Anspruch 1 definiert ist, bei der Behandlung von Krebs.

## Revendications

**1.** Composition pharmaceutique comprenant un composé de type sulfonamide, en combinaison avec une substance dotée d’une activité d’inhibition du facteur de croissance EGF,
dans laquelle le composé de type sulfonamide est du N-(3-chloro-1H-indol-7-yl)-4-sulfamyl-benzène-sulfonamide, du N-(3-cyano-4-méthyl-1H-indol-7-yl)-3-cyano-benzène-sulfonamide, ou un sel pharmacologiquement admissible ou un solvat de l’un de ces composés,
et dans laquelle la substance dotée d’une activité d’inhibition de l’EGF est du géfitinib, de l’erlotinib ou du cétuximab.

**2.** Composition pharmaceutique conforme à la revendication 1, laquelle composition pharmaceutique est utilisée pour traiter un cancer.

**3.** Trousse comprenant un assortiment d’une formulation comprenant un composé de type sulfonamide, défini dans

la revendication 1, et d'une formulation comprenant une substance dotée d'une activité d'inhibition de l'EGF, définie dans la revendication 1.

4. Trousse conforme à la revendication 4, pour utilisation dans le traitement d'un cancer.

5. Utilisation d'un composé de type sulfonamide, défini dans la revendication 1, en vue de produire une composition pharmaceutique pour utilisation, en combinaison avec une substance dotée d'une activité d'inhibition de l'EGF, définie dans la revendication 1, dans le traitement d'un cancer.

6. Utilisation d'une substance dotée d'une activité d'inhibition de l'EGF, définie dans la revendication 1, en vue de produire une composition pharmaceutique pour utilisation, en combinaison avec un composé de type sulfonamide, défini dans la revendication 1, dans le traitement d'un cancer.

7. Composé de type sulfonamide, défini dans la revendication 1, pour utilisation, en combinaison avec une substance dotée d'une activité d'inhibition de l'EGF, définie dans la revendication 1, dans le traitement d'un cancer.

8. Substance dotée d'une activité d'inhibition de l'EGF, définie dans la revendication 1, pour utilisation, en combinaison avec un composé de type sulfonamide, défini dans la revendication 1, dans le traitement d'un cancer.

9. Composition pharmaceutique comprenant un composé de type sulfonamide, défini dans la revendication 1, pour utilisation, en combinaison avec une substance dotée d'une activité d'inhibition de l'EGF, définie dans la revendication 1, dans le traitement d'un cancer.

10. Composition pharmaceutique comprenant une substance dotée d'une activité d'inhibition de l'EGF, définie dans la revendication 1, pour utilisation, en combinaison avec un composé de type sulfonamide, défini dans la revendication 1, dans le traitement d'un cancer.

Figure 1

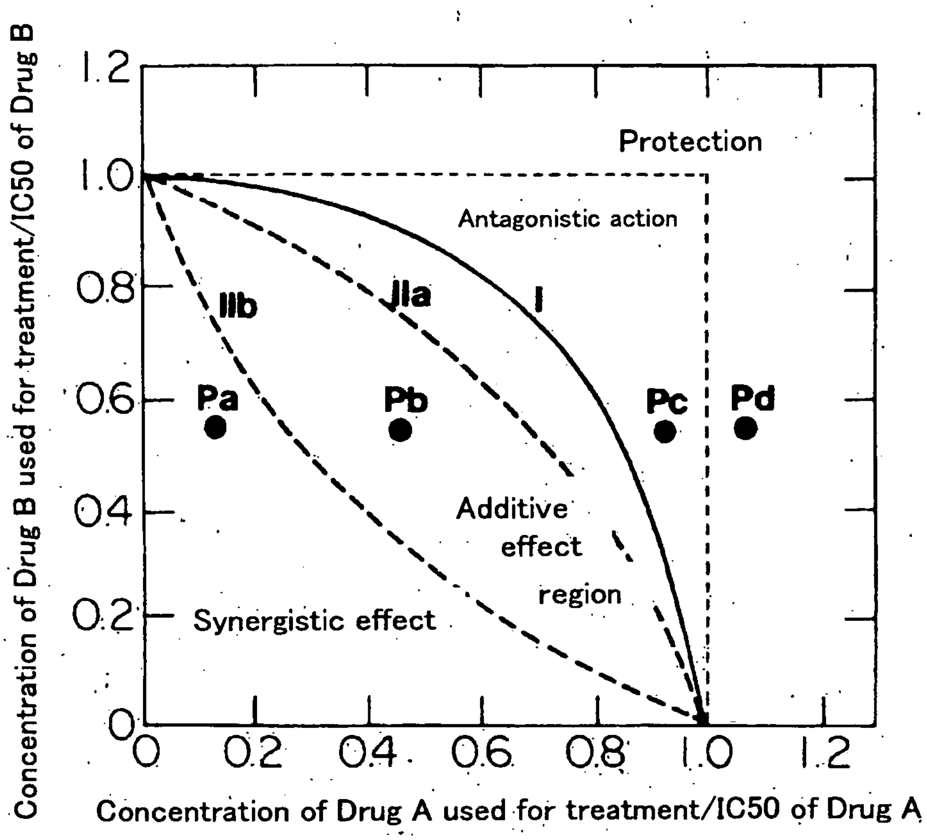
Concentration of Drug B used for treatment/IC50 of Drug B
1.2
1.0
0.8
0.6
0.4
0.2
0
Protection
Antagonistic action
IIb
IIa
I
Pa
Pb
Pc
Pd
Additive effect region
Synergistic effect
0
0.2
0.4
0.6
0.8
1.0
1.2
Concentration of Drug A used for treatment/IC50 of Drug A

Figure 2

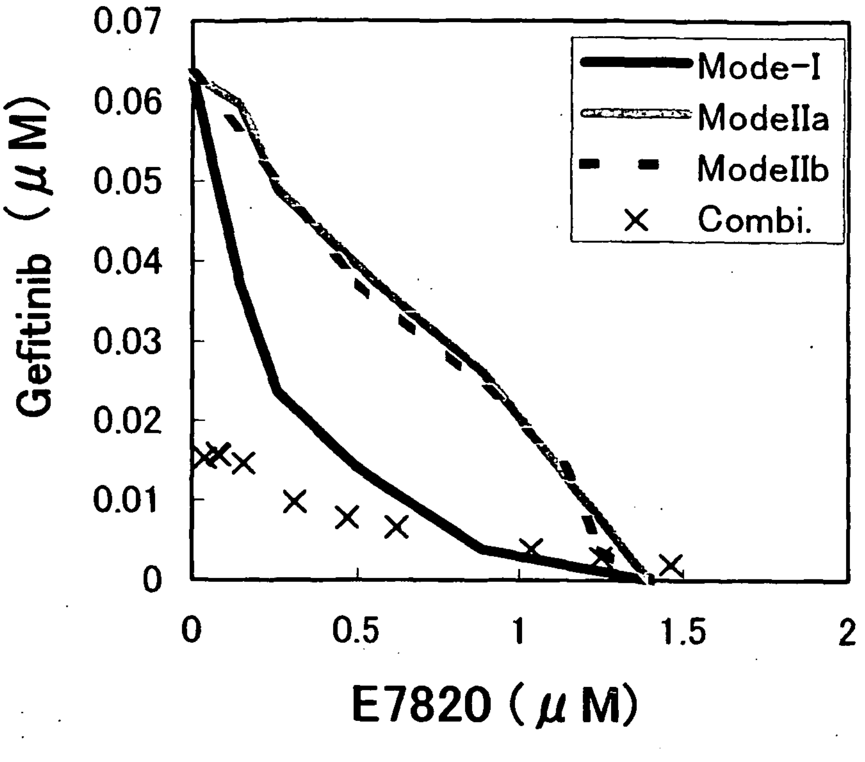
0.07
0.06
0.05
0.04
0.03
0.02
0.01
0
0
0.5
1
1.5
2
Gefitinib (μM)
E7820 (μM)
Mode-I
ModeIIa
ModeIIb
Combi.

Figure 3

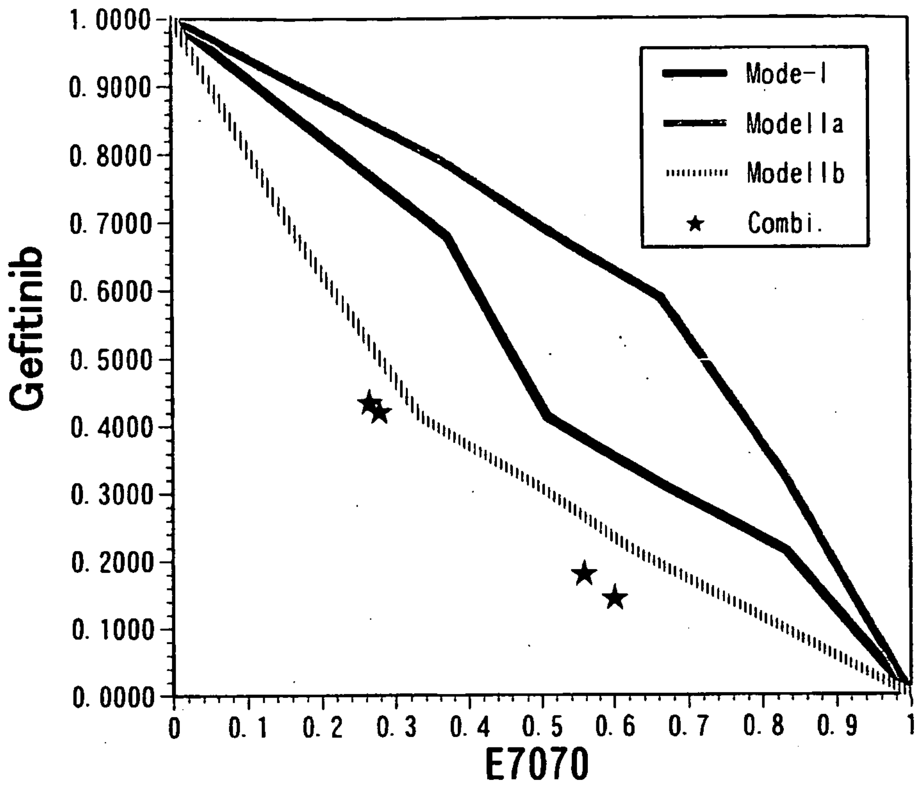
1. 0000
0. 9000
0. 8000
0. 7000
0. 6000
0. 5000
0. 4000
0. 3000
0. 2000
0. 1000
0. 0000
Gefitinib
0
0. 1
0. 2
0. 3
0. 4
0. 5
0. 6
0. 7
0. 8
0. 9
1
E7070
Mode-I
ModelIa
ModelIb
Combi.

Figure 4

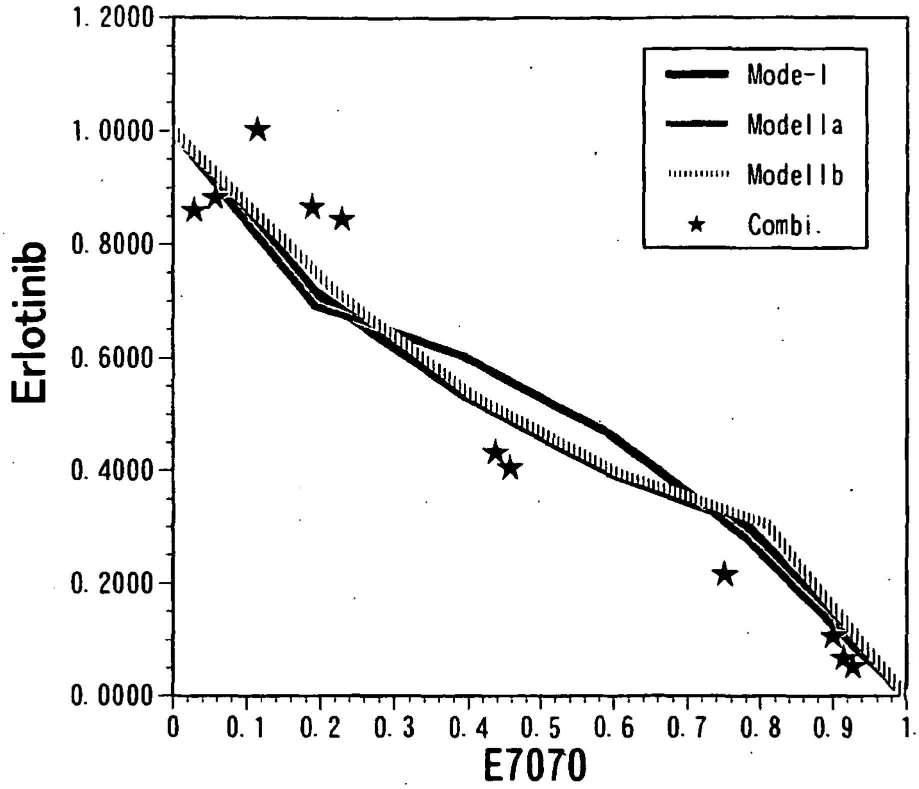
1. 2000
1. 0000
0. 8000
0. 6000
0. 4000
0. 2000
0. 0000
Erlotinib
0
0. 1
0. 2
0. 3
0. 4
0. 5
0. 6
0. 7
0. 8
0. 9
1
E7070
Mode-I
ModelIa
ModelIb
Combi.

Figure 5

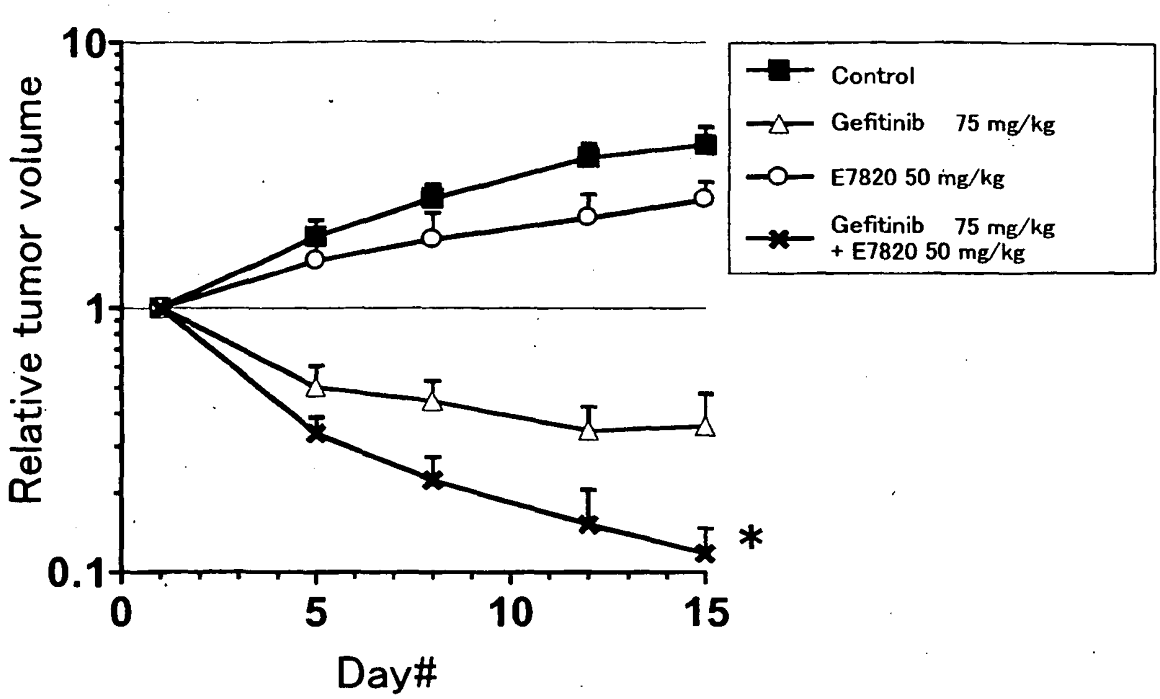
10
1
0.1
Relative tumor volume
0
5
10
15
Day#
Control
Gefitinib 75 mg/kg
E7820 50 mg/kg
Gefitinib 75 mg/kg
+ E7820 50 mg/kg
*

Figure 6

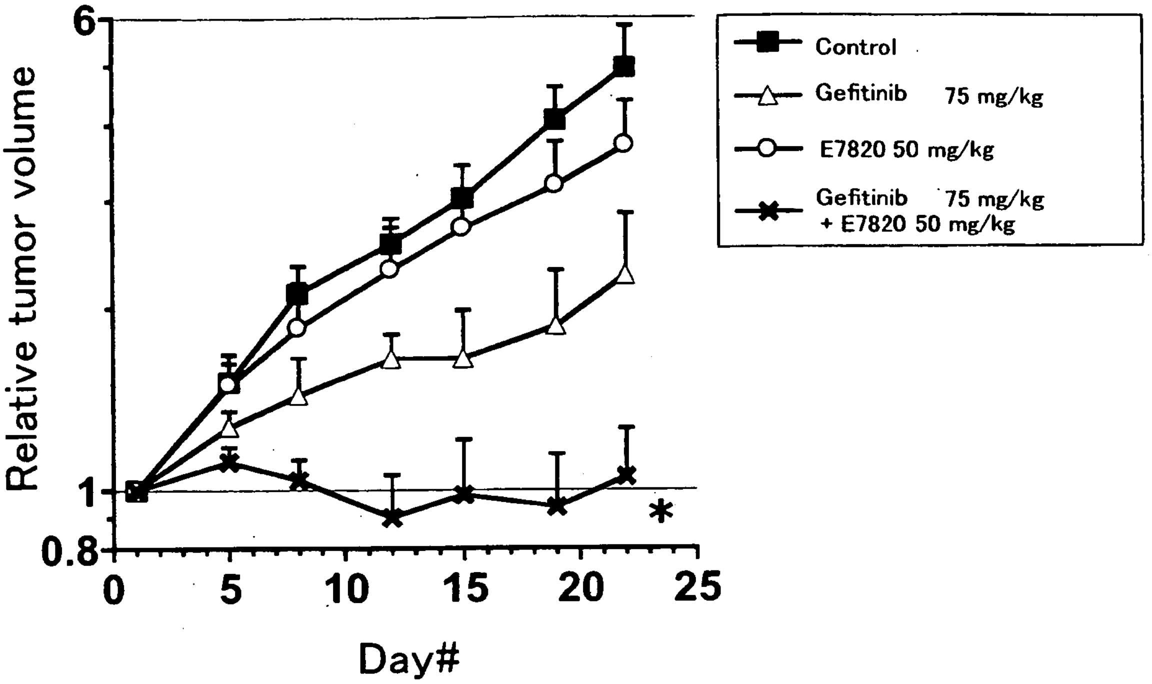
6
1
0.8
0
5
10
15
20
25
Relative tumor volume
Day#
Control
Gefitinib 75 mg/kg
E7820 50 mg/kg
Gefitinib 75 mg/kg
+ E7820 50 mg/kg
*

Figure 7

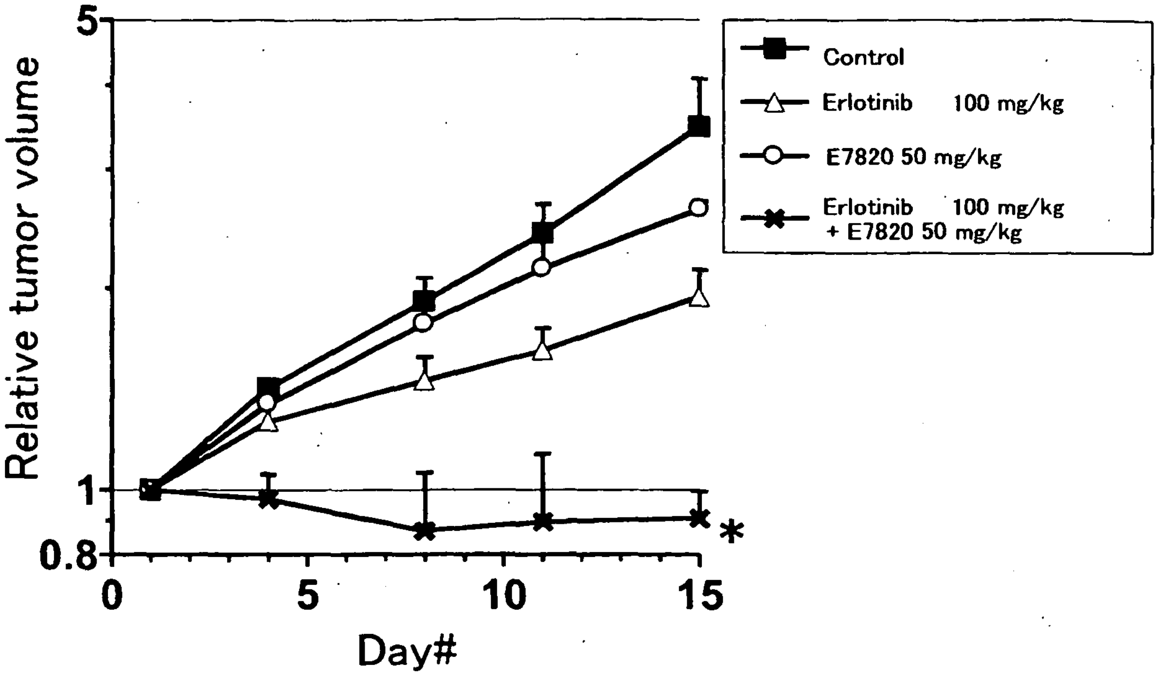
Control
Erlotinib 100 mg/kg
E7820 50 mg/kg
Erlotinib 100 mg/kg
+ E7820 50 mg/kg
Relative tumor volume
5
1
0.8
0
5
10
15
Day#
*

Figure 8

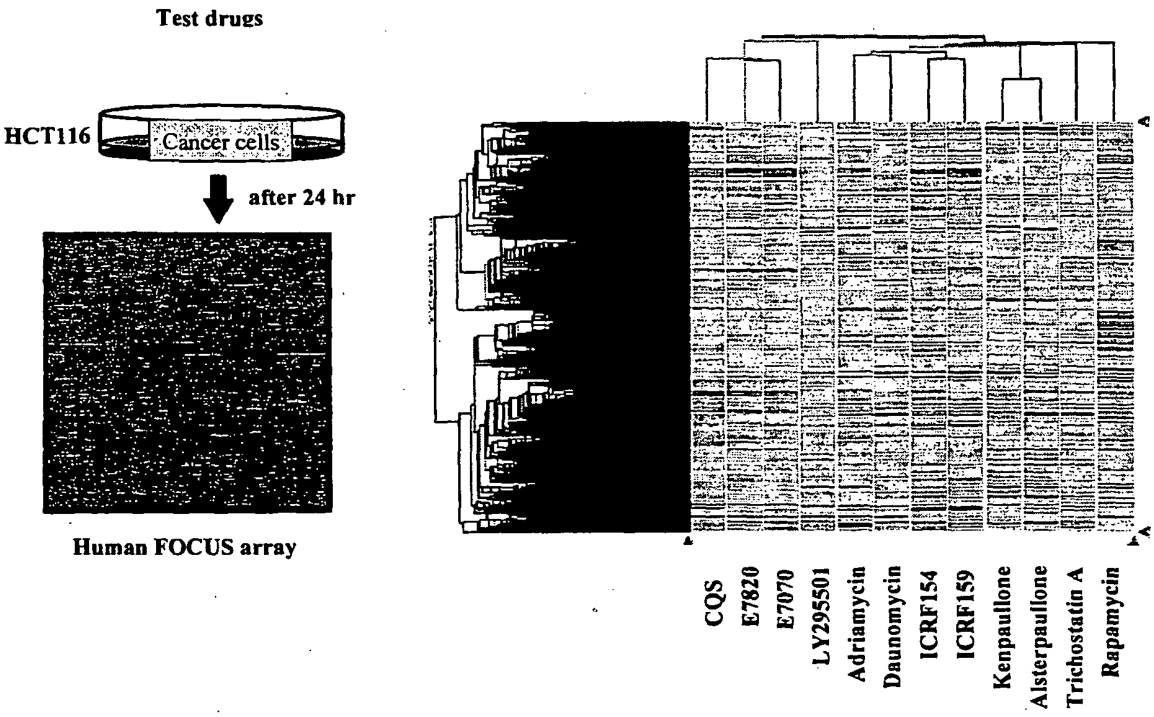
Test drugs
HCT116
Cancer cells
after 24 hr
Human FOCUS array
CQS
E7820
E7070
LY295501
Adriamycin
Daunomycin
ICRF154
ICRF159
Kenpaullone
Alsterpaullone
Trichostatin A
Rapamycin

Figure 9

| | E7070-1 | E7070-2 | E7820-1 | E7820-2 | CQS-1 | CQS-2 | LY1-1 | LY1-2 | LY2-1 | LY2-2 | LY5-1 | LY5-2 | CAI-1 | CAI-2 | Cap-1 | Cap-2 | MST-1 | MST-2 | Etop-1 | Etop-2 | TSA-1 | TSA-2 | Kenp-1 | Kenp-2 |
|---|---|---|---|---|---|---|---|---|---|---|---|---|---|---|---|---|---|---|---|---|---|---|---|---|
| E7070-1 | *1.00* | 0.95 | 0.87 | 0.87 | 0.87 | 0.87 | 0.83 | 0.83 | 0.86 | 0.86 | 0.88 | 0.88 | 0.16 | 0.17 | 0.11 | 0.11 | 0.35 | 0.35 | 0.16 | 0.17 | 0.31 | 0.31 | 0.32 | 0.32 |
| E7070-2 | 0.95 | *1.00* | 0.87 | 0.86 | 0.87 | 0.87 | 0.83 | 0.83 | 0.86 | 0.85 | 0.88 | 0.88 | 0.17 | 0.17 | 0.11 | 0.11 | 0.36 | 0.36 | 0.16 | 0.17 | 0.32 | 0.32 | 0.32 | 0.33 |
| E7820-1 | 0.87 | 0.87 | *1.00* | 0.96 | 0.90 | 0.90 | 0.88 | 0.88 | 0.83 | 0.82 | 0.88 | 0.88 | 0.09 | 0.09 | 0.07 | 0.07 | 0.26 | 0.26 | 0.08 | 0.08 | 0.24 | 0.24 | 0.20 | 0.21 |
| E7820-2 | 0.87 | 0.86 | 0.96 | *1.00* | 0.90 | 0.90 | 0.88 | 0.88 | 0.83 | 0.82 | 0.88 | 0.88 | 0.09 | 0.09 | 0.07 | 0.07 | 0.27 | 0.26 | 0.08 | 0.09 | 0.24 | 0.25 | 0.21 | 0.21 |
| CQS-1 | 0.87 | 0.87 | 0.90 | 0.90 | *1.00* | 0.96 | 0.87 | 0.87 | 0.84 | 0.83 | 0.90 | 0.90 | 0.13 | 0.14 | 0.10 | 0.10 | 0.37 | 0.36 | 0.16 | 0.16 | 0.32 | 0.32 | 0.29 | 0.29 |
| CQS-2 | 0.87 | 0.87 | 0.90 | 0.90 | 0.96 | *1.00* | 0.87 | 0.87 | 0.84 | 0.83 | 0.90 | 0.90 | 0.14 | 0.14 | 0.10 | 0.10 | 0.37 | 0.37 | 0.16 | 0.16 | 0.32 | 0.33 | 0.29 | 0.29 |
| LY1-1 | 0.83 | 0.83 | 0.88 | 0.88 | 0.87 | 0.87 | *1.00* | 0.97 | 0.82 | 0.81 | 0.89 | 0.89 | 0.25 | 0.25 | 0.24 | 0.24 | 0.30 | 0.30 | 0.12 | 0.13 | 0.25 | 0.26 | 0.25 | 0.25 |
| LY1-2 | 0.83 | 0.83 | 0.88 | 0.88 | 0.87 | 0.87 | 0.97 | *1.00* | 0.82 | 0.81 | 0.89 | 0.88 | 0.25 | 0.25 | 0.24 | 0.25 | 0.30 | 0.30 | 0.12 | 0.13 | 0.25 | 0.26 | 0.25 | 0.26 |
| LY2-1 | 0.86 | 0.86 | 0.83 | 0.83 | 0.84 | 0.84 | 0.82 | 0.82 | *1.00* | 0.94 | 0.85 | 0.85 | 0.20 | 0.20 | 0.14 | 0.15 | 0.35 | 0.35 | 0.17 | 0.18 | 0.34 | 0.34 | 0.32 | 0.32 |
| LY2-2 | 0.86 | 0.85 | 0.82 | 0.82 | 0.83 | 0.83 | 0.81 | 0.81 | 0.94 | *1.00* | 0.84 | 0.84 | 0.19 | 0.19 | 0.15 | 0.15 | 0.35 | 0.35 | 0.18 | 0.18 | 0.33 | 0.34 | 0.31 | 0.32 |
| LY5-1 | 0.88 | 0.88 | 0.88 | 0.88 | 0.90 | 0.90 | 0.89 | 0.89 | 0.85 | 0.84 | *1.00* | 0.97 | 0.20 | 0.20 | 0.15 | 0.15 | 0.39 | 0.39 | 0.21 | 0.22 | 0.34 | 0.34 | 0.31 | 0.32 |
| LY5-2 | 0.88 | 0.88 | 0.88 | 0.88 | 0.90 | 0.90 | 0.89 | 0.88 | 0.85 | 0.84 | 0.97 | *1.00* | 0.20 | 0.20 | 0.15 | 0.15 | 0.39 | 0.39 | 0.21 | 0.22 | 0.34 | 0.34 | 0.31 | 0.32 |
| CAI-1 | 0.16 | 0.17 | 0.09 | 0.09 | 0.13 | 0.14 | 0.25 | 0.25 | 0.20 | 0.19 | 0.20 | 0.20 | *1.00* | 0.98 | 0.75 | 0.75 | 0.38 | 0.38 | 0.34 | 0.34 | 0.25 | 0.25 | 0.25 | 0.25 |
| CAI-2 | 0.17 | 0.17 | 0.09 | 0.09 | 0.14 | 0.14 | 0.25 | 0.25 | 0.20 | 0.19 | 0.20 | 0.20 | 0.98 | *1.00* | 0.75 | 0.75 | 0.38 | 0.38 | 0.34 | 0.33 | 0.25 | 0.25 | 0.26 | 0.26 |
| Cap-1 | 0.11 | 0.11 | 0.07 | 0.07 | 0.10 | 0.10 | 0.24 | 0.24 | 0.14 | 0.15 | 0.15 | 0.15 | 0.75 | 0.75 | *1.00* | 0.99 | 0.35 | 0.34 | 0.31 | 0.31 | 0.19 | 0.19 | 0.14 | 0.14 |
| Cap-2 | 0.11 | 0.11 | 0.07 | 0.07 | 0.10 | 0.10 | 0.24 | 0.25 | 0.15 | 0.15 | 0.15 | 0.15 | 0.75 | 0.75 | 0.99 | *1.00* | 0.34 | 0.34 | 0.31 | 0.31 | 0.18 | 0.18 | 0.14 | 0.14 |
| MST-1 | 0.35 | 0.36 | 0.26 | 0.27 | 0.37 | 0.37 | 0.30 | 0.30 | 0.35 | 0.35 | 0.39 | 0.39 | 0.38 | 0.38 | 0.35 | 0.34 | *1.00* | 0.94 | 0.62 | 0.62 | 0.57 | 0.57 | 0.39 | 0.39 |
| MST-2 | 0.35 | 0.36 | 0.26 | 0.26 | 0.36 | 0.37 | 0.30 | 0.30 | 0.35 | 0.35 | 0.39 | 0.39 | 0.38 | 0.38 | 0.34 | 0.34 | 0.94 | *1.00* | 0.62 | 0.63 | 0.57 | 0.57 | 0.39 | 0.39 |
| Etop-1 | 0.16 | 0.16 | 0.08 | 0.08 | 0.16 | 0.16 | 0.12 | 0.12 | 0.17 | 0.18 | 0.21 | 0.21 | 0.34 | 0.34 | 0.31 | 0.31 | 0.62 | 0.62 | *1.00* | 0.96 | 0.45 | 0.45 | 0.24 | 0.24 |
| Etop-2 | 0.17 | 0.17 | 0.08 | 0.09 | 0.16 | 0.16 | 0.13 | 0.13 | 0.18 | 0.18 | 0.22 | 0.22 | 0.34 | 0.33 | 0.31 | 0.31 | 0.62 | 0.63 | 0.96 | *1.00* | 0.45 | 0.45 | 0.24 | 0.24 |
| TSA-1 | 0.31 | 0.32 | 0.24 | 0.24 | 0.32 | 0.32 | 0.25 | 0.25 | 0.34 | 0.33 | 0.34 | 0.34 | 0.25 | 0.25 | 0.19 | 0.18 | 0.57 | 0.57 | 0.45 | 0.45 | *1.00* | 0.93 | 0.34 | 0.34 |
| TSA-2 | 0.31 | 0.32 | 0.24 | 0.25 | 0.32 | 0.33 | 0.26 | 0.26 | 0.34 | 0.34 | 0.34 | 0.34 | 0.25 | 0.25 | 0.19 | 0.18 | 0.57 | 0.57 | 0.45 | 0.45 | 0.93 | *1.00* | 0.34 | 0.34 |
| Kenp-1 | 0.32 | 0.32 | 0.20 | 0.21 | 0.29 | 0.29 | 0.25 | 0.25 | 0.32 | 0.31 | 0.31 | 0.31 | 0.25 | 0.26 | 0.14 | 0.14 | 0.39 | 0.39 | 0.24 | 0.24 | 0.34 | 0.34 | *1.00* | 0.96 |
| Kenp-2 | 0.32 | 0.33 | 0.21 | 0.21 | 0.29 | 0.29 | 0.25 | 0.26 | 0.32 | 0.32 | 0.32 | 0.32 | 0.25 | 0.26 | 0.14 | 0.14 | 0.39 | 0.39 | 0.24 | 0.24 | 0.34 | 0.34 | 0.96 | *1.00* |

0.7<X<=1.0

0.4<X<=0.7

0<X<=0.4

Figure 10

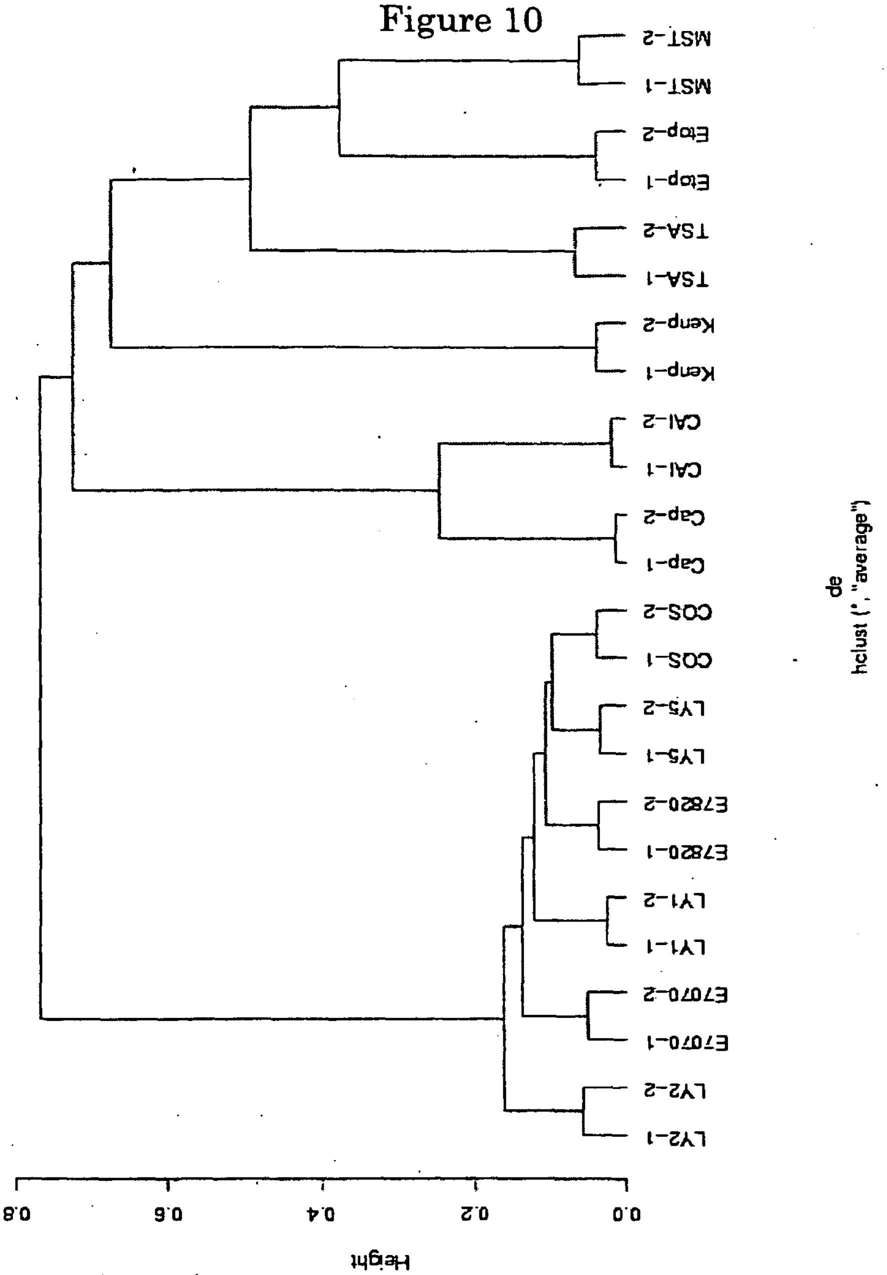
MST-2
MST-1
Etop-2
Etop-1
TSA-2
TSA-1
Kenp-2
Kenp-1
CAI-2
CAI-1
Cap-2
Cap-1
COS-2
COS-1
LY5-2
LY5-1
E7820-2
E7820-1
LY1-2
LY1-1
E7070-2
E7070-1
LY2-2
LY2-1
0.0
0.2
0.4
0.6
0.8
Height
de
hclust (*, "average")

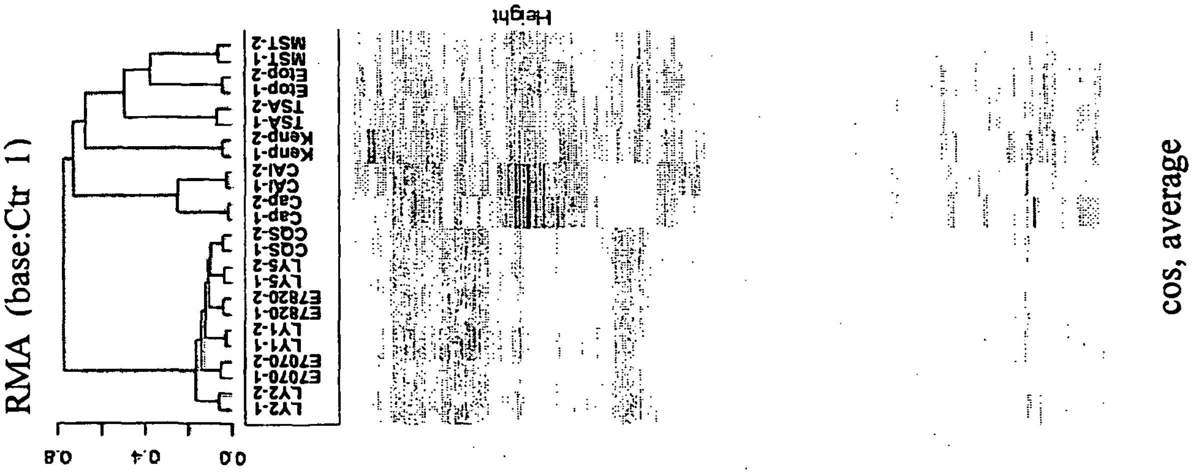
RMA (base:Ctr 1)
cos, average

# Figure 11

| | E7070-1 | E7070-2 | E7820-1 | E7820-2 | CQS-1 | CQS-2 | LY1-1 | LY1-2 | LY2-1 | LY2-2 | LY5-1 | LY5-2 | CAI-1 | CAI-2 | Cap-1 | Cap-2 | MST-1 | MST-2 | Etop-1 | Etop-2 | TSA-1 | TSA-2 | Kenp-1 | Kenp-2 |
|---|---|---|---|---|---|---|---|---|---|---|---|---|---|---|---|---|---|---|---|---|---|---|---|---|
| E7070-1 | *1.00* | 0.94 | 0.87 | 0.87 | 0.86 | 0.86 | 0.81 | 0.81 | 0.84 | 0.83 | 0.86 | 0.86 | 0.04 | 0.04 | 0.03 | 0.04 | 0.24 | 0.23 | 0.06 | 0.06 | 0.22 | 0.22 | 0.19 | 0.19 |
| E7070-2 | 0.94 | *1.00* | 0.86 | 0.86 | 0.85 | 0.85 | 0.81 | 0.81 | 0.84 | 0.83 | 0.86 | 0.86 | 0.04 | 0.04 | 0.03 | 0.03 | 0.24 | 0.23 | 0.05 | 0.06 | 0.22 | 0.22 | 0.19 | 0.20 |
| E7820-1 | 0.87 | 0.86 | *1.00* | 0.96 | 0.90 | 0.90 | 0.86 | 0.86 | 0.81 | 0.80 | 0.87 | 0.87 | 0.00 | 0.00 | 0.02 | 0.03 | 0.22 | 0.22 | 0.03 | 0.04 | 0.21 | 0.22 | 0.12 | 0.12 |
| E7820-2 | 0.87 | 0.86 | 0.96 | *1.00* | 0.90 | 0.90 | 0.85 | 0.85 | 0.81 | 0.80 | 0.87 | 0.87 | 0.00 | 0.00 | 0.02 | 0.03 | 0.22 | 0.21 | 0.03 | 0.04 | 0.21 | 0.22 | 0.12 | 0.13 |
| CQS-1 | 0.86 | 0.85 | 0.90 | 0.90 | *1.00* | 0.96 | 0.85 | 0.85 | 0.82 | 0.81 | 0.89 | 0.88 | 0.02 | 0.02 | 0.03 | 0.03 | 0.28 | 0.27 | 0.07 | 0.07 | 0.25 | 0.25 | 0.17 | 0.18 |
| CQS-2 | 0.86 | 0.85 | 0.90 | 0.90 | 0.96 | *1.00* | 0.86 | 0.85 | 0.82 | 0.81 | 0.89 | 0.89 | 0.02 | 0.02 | 0.03 | 0.04 | 0.28 | 0.27 | 0.07 | 0.07 | 0.25 | 0.25 | 0.17 | 0.18 |
| LY1-1 | 0.81 | 0.81 | 0.86 | 0.85 | 0.85 | 0.86 | *1.00* | 0.97 | 0.80 | 0.79 | 0.88 | 0.88 | 0.17 | 0.17 | 0.19 | 0.19 | 0.20 | 0.20 | 0.04 | 0.04 | 0.17 | 0.17 | 0.15 | 0.16 |
| LY1-2 | 0.81 | 0.81 | 0.86 | 0.85 | 0.85 | 0.85 | 0.97 | *1.00* | 0.80 | 0.79 | 0.87 | 0.87 | 0.17 | 0.17 | 0.19 | 0.20 | 0.20 | 0.20 | 0.03 | 0.04 | 0.17 | 0.17 | 0.15 | 0.16 |
| LY2-1 | 0.84 | 0.84 | 0.81 | 0.81 | 0.82 | 0.82 | 0.80 | 0.80 | *1.00* | 0.93 | 0.83 | 0.83 | 0.09 | 0.09 | 0.07 | 0.08 | 0.23 | 0.23 | 0.07 | 0.08 | 0.24 | 0.24 | 0.20 | 0.20 |
| LY2-2 | 0.83 | 0.83 | 0.80 | 0.80 | 0.81 | 0.81 | 0.79 | 0.79 | 0.93 | *1.00* | 0.82 | 0.82 | 0.08 | 0.08 | 0.07 | 0.08 | 0.23 | 0.22 | 0.07 | 0.07 | 0.23 | 0.24 | 0.19 | 0.20 |
| LY5-1 | 0.86 | 0.86 | 0.87 | 0.87 | 0.89 | 0.89 | 0.88 | 0.87 | 0.83 | 0.82 | *1.00* | 0.96 | 0.09 | 0.09 | 0.08 | 0.08 | 0.28 | 0.28 | 0.11 | 0.12 | 0.24 | 0.24 | 0.19 | 0.20 |
| LY5-2 | 0.86 | 0.86 | 0.87 | 0.87 | 0.88 | 0.89 | 0.88 | 0.87 | 0.83 | 0.82 | 0.96 | *1.00* | 0.09 | 0.09 | 0.07 | 0.08 | 0.28 | 0.28 | 0.11 | 0.12 | 0.24 | 0.24 | 0.19 | 0.20 |
| CAI-1 | 0.04 | 0.04 | 0.00 | 0.00 | 0.02 | 0.02 | 0.17 | 0.17 | 0.09 | 0.08 | 0.09 | 0.09 | *1.00* | 0.98 | 0.74 | 0.74 | 0.26 | 0.26 | 0.25 | 0.25 | 0.13 | 0.13 | 0.14 | 0.14 |
| CAI-2 | 0.04 | 0.04 | 0.00 | 0.00 | 0.02 | 0.02 | 0.17 | 0.17 | 0.09 | 0.08 | 0.09 | 0.09 | 0.98 | *1.00* | 0.74 | 0.74 | 0.26 | 0.26 | 0.25 | 0.25 | 0.13 | 0.13 | 0.15 | 0.15 |
| Cap-1 | 0.03 | 0.03 | 0.02 | 0.02 | 0.03 | 0.03 | 0.19 | 0.19 | 0.07 | 0.07 | 0.08 | 0.07 | 0.74 | 0.74 | *1.00* | 0.99 | 0.28 | 0.28 | 0.26 | 0.26 | 0.12 | 0.12 | 0.06 | 0.06 |
| Cap-2 | 0.04 | 0.03 | 0.03 | 0.03 | 0.03 | 0.04 | 0.19 | 0.20 | 0.08 | 0.08 | 0.08 | 0.08 | 0.74 | 0.74 | 0.99 | *1.00* | 0.28 | 0.28 | 0.26 | 0.25 | 0.11 | 0.12 | 0.06 | 0.06 |
| MST-1 | 0.24 | 0.24 | 0.22 | 0.22 | 0.28 | 0.28 | 0.20 | 0.20 | 0.23 | 0.23 | 0.28 | 0.28 | 0.26 | 0.26 | 0.28 | 0.28 | *1.00* | 0.92 | 0.57 | 0.57 | 0.50 | 0.50 | 0.24 | 0.25 |
| MST-2 | 0.23 | 0.23 | 0.22 | 0.21 | 0.27 | 0.27 | 0.20 | 0.20 | 0.23 | 0.22 | 0.28 | 0.28 | 0.26 | 0.26 | 0.28 | 0.28 | 0.92 | *1.00* | 0.57 | 0.57 | 0.50 | 0.50 | 0.24 | 0.25 |
| Etop-1 | 0.06 | 0.05 | 0.03 | 0.03 | 0.07 | 0.07 | 0.04 | 0.03 | 0.07 | 0.07 | 0.11 | 0.11 | 0.25 | 0.25 | 0.26 | 0.26 | 0.57 | 0.57 | *1.00* | 0.96 | 0.39 | 0.39 | 0.12 | 0.13 |
| Etop-2 | 0.06 | 0.06 | 0.04 | 0.04 | 0.07 | 0.07 | 0.04 | 0.04 | 0.08 | 0.07 | 0.12 | 0.12 | 0.25 | 0.25 | 0.26 | 0.25 | 0.57 | 0.57 | 0.96 | *1.00* | 0.39 | 0.39 | 0.12 | 0.13 |
| TSA-1 | 0.22 | 0.22 | 0.21 | 0.21 | 0.25 | 0.25 | 0.17 | 0.17 | 0.24 | 0.23 | 0.24 | 0.24 | 0.13 | 0.13 | 0.12 | 0.11 | 0.50 | 0.50 | 0.39 | 0.39 | *1.00* | 0.93 | 0.21 | 0.22 |
| TSA-2 | 0.22 | 0.22 | 0.22 | 0.22 | 0.25 | 0.25 | 0.17 | 0.17 | 0.24 | 0.24 | 0.24 | 0.24 | 0.13 | 0.13 | 0.12 | 0.12 | 0.50 | 0.50 | 0.39 | 0.39 | 0.93 | *1.00* | 0.22 | 0.22 |
| Kenp-1 | 0.19 | 0.19 | 0.12 | 0.12 | 0.17 | 0.17 | 0.15 | 0.15 | 0.20 | 0.19 | 0.19 | 0.19 | 0.14 | 0.15 | 0.06 | 0.06 | 0.24 | 0.24 | 0.12 | 0.12 | 0.21 | 0.22 | *1.00* | 0.95 |
| Kenp-2 | 0.19 | 0.20 | 0.12 | 0.13 | 0.18 | 0.18 | 0.16 | 0.16 | 0.20 | 0.20 | 0.20 | 0.20 | 0.14 | 0.15 | 0.06 | 0.06 | 0.25 | 0.25 | 0.13 | 0.13 | 0.22 | 0.22 | 0.95 | *1.00* |

$0.7 < X \leq 1.0$

$0.4 < X \leq 0.7$

$0 < X \leq 0.4$

Figure 12

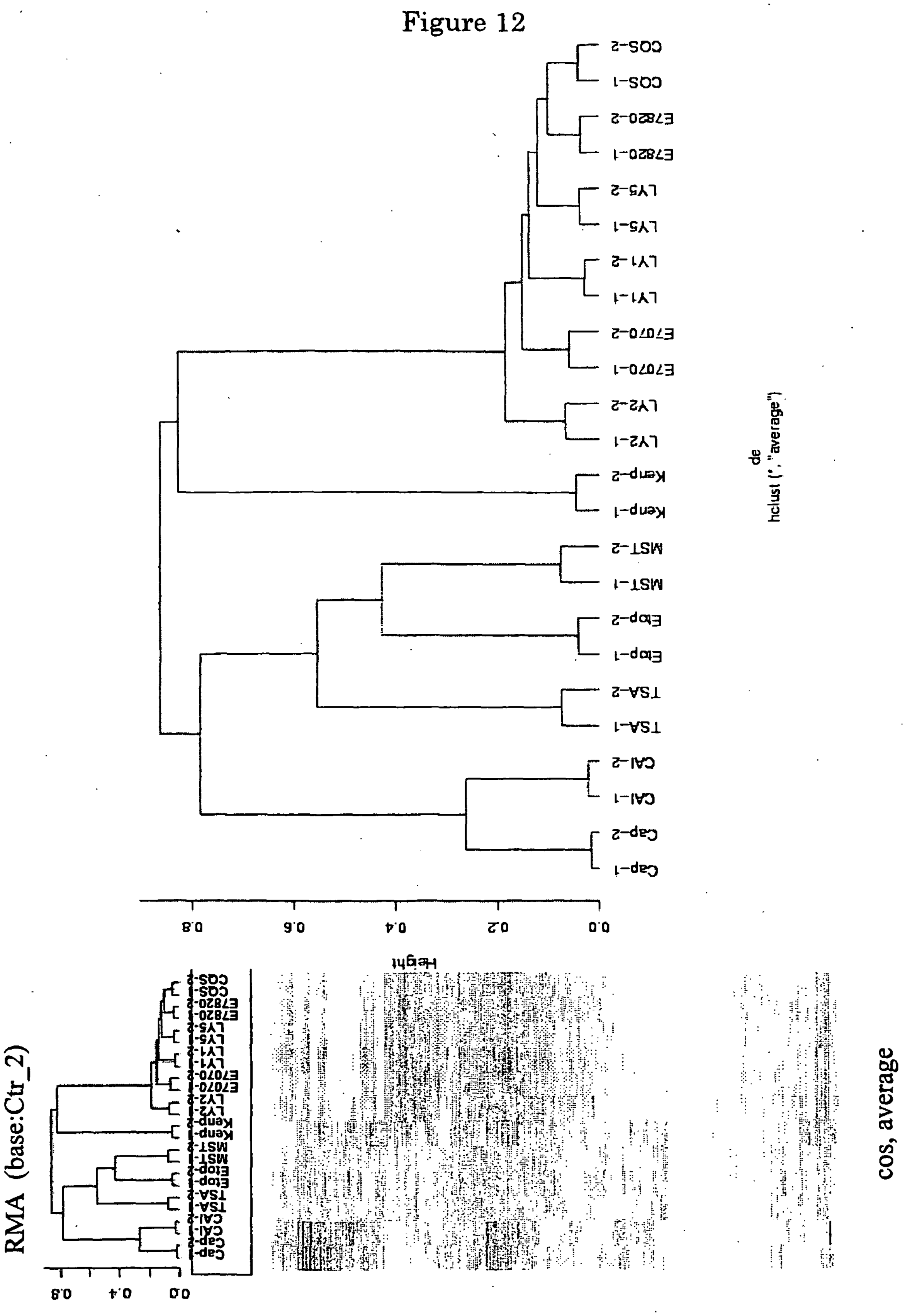
COS-2
COS-1
E7820-2
E7820-1
LY5-2
LY5-1
LY1-2
LY1-1
E7070-2
E7070-1
LY2-2
LY2-1
Kenp-2
Kenp-1
MST-2
MST-1
Etop-2
Etop-1
TSA-2
TSA-1
CAI-2
CAI-1
Cap-2
Cap-1
0.0
0.2
0.4
0.6
0.8
Height
de
hclust (*, "average")
RMA (base:Ctr_2)
cos, average

Figure 13

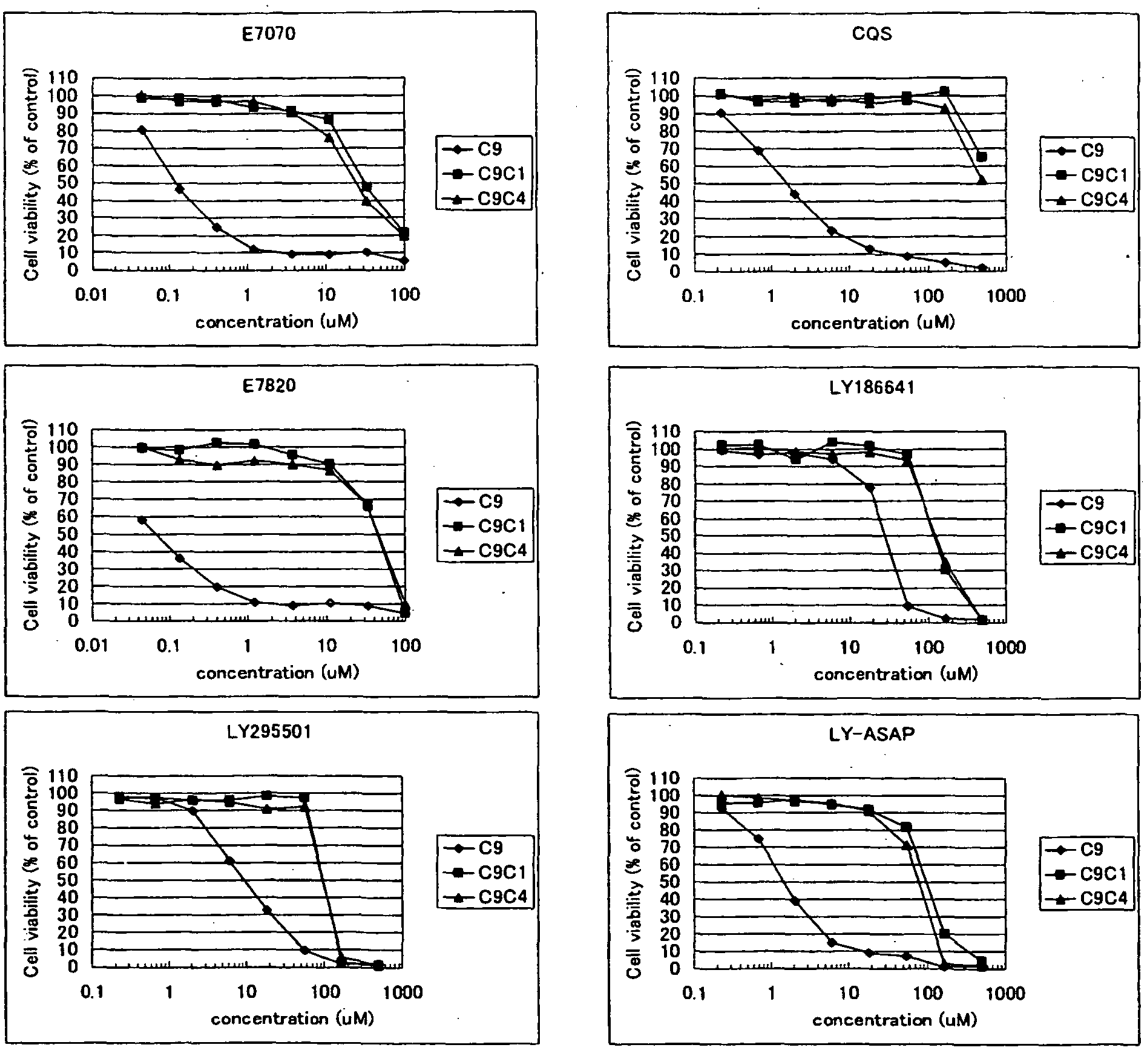
E7070
CQS
E7820
LY186641
LY295501
LY-ASAP
Cell viability (% of control)
concentration (uM)
C9
C9C1
C9C4

Figure 14

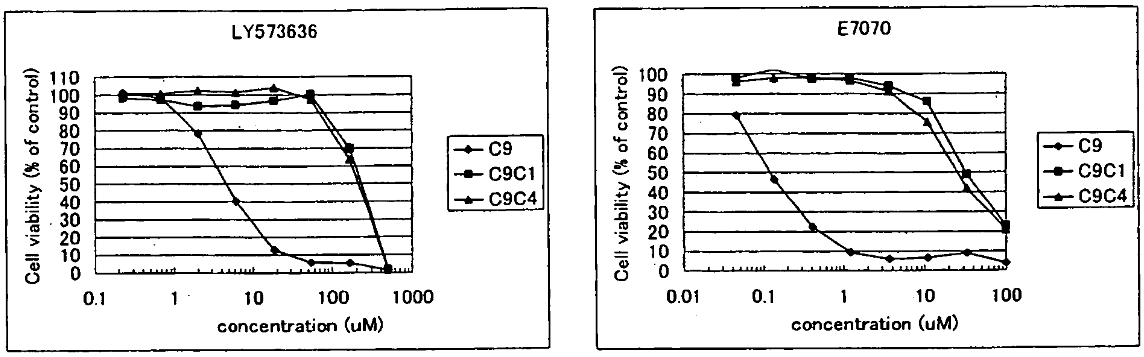
LY573636
Cell viability (% of control)
110
100
90
80
70
60
50
40
30
20
10
0
0.1
1
10
100
1000
concentration (uM)
C9
C9C1
C9C4
E7070
Cell viability (% of control)
100
90
80
70
60
50
40
30
20
10
0
0.01
0.1
1
10
100
concentration (uM)
C9
C9C1
C9C4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7165708 A
- WO 0050395 A
- EP 0222475 A
- WO 02098848 A
- WO 2003035629 A
- WO 9633980 A
- JP 3040486 B
- JP 3088018 B
- JP 2291295 A
- WO 9507276 A
- US 5770599 A
- WO 9630347 A
- JP 3420549 B
- JP 2002114710 A
- JP 2004114710 A


### Non-patent literature cited in the description


- **Schena M ; Shalon D ; Davis RW ; Brown PO.** *Science,* 1995, vol. 270, 467-70
- **Lockhart, D.J. ; Dong, H. ; Byrne, M.C. ; Follettie, M.T. ; Gallo, M.V. ; Chee, M.S. ; Mittmann, M. ; Wang C. ; Kobayashi, M. ; Horton, H.** *Nature Biotechnology,* 1996, vol. 14, 1675-1680
- **Rhee CH ; Ruan S ; Chen S ; Chenchik A ; Levin VA ; Yung AW ; Fuller GN ; Zhang W.** *Oncol Rep,* 1999, vol. 6, 393-401
- **Zimmermann J ; Erdmann D ; Lalande I ; Grossenbacher R ; Noorani M ; Furst P.** *Oncogene,* 2000, vol. 19, 2913-20
- **Kudoh K ; Ramanna M ; Ravatn R ; Elkahloun AG ; Bittner ML ; Meltzer PS ; Trent JM ; Dalton WS ; Chin KV.** *Cancer Res,* 2000, 4161-6
- **Ross DT ; Scherf U ; Eisen MB ; Perou CM ; Rees C ; Spellman P ; Iyer V ; Jeffrey SS ; Van de Rijn M ; Waltham M.** *Nat Genet,* 2000, vol. 24, 227-35
- **Scherf U ; Ross DT ; Waltham M ; Smith LH ; Lee JK ; Tanabe L ; Kohn KW ; Reinhold WC ; Myers TG ; Andrews DT.** *Nat Genet,* 2000, vol. 24, 236-44
- **Steel GG et al.** *Int J Radiat Oncol Biol Phys,* 1979, vol. 5, 85-91
- **Kano Y et al.** *Int J Cancer,* 1992, vol. 50, 604-610
- *Biostatistics,* 2003, vol. 4, 249-264
- **Paull, K. D. et al.** Display and analysis of patterns of differential activity of drugs against human tumor cell lines: development of mean graph and COMPARE algorithm. *J. Natl. Cancer Inst.,* 1989, vol. 81, 1088-1092
- **Monks, A. et al.** Feasibility of a high-flux anticancer drug screen using a diverse panel of cultured human tumor cell lines. *J. Natl. Cancer Inst.,* 1991, vol. 83, 757-766
- *Molecular Cancer Therapeutics,* 2002, vol. 1, 275-286
- **Mossmann T.** *J. Immunol. Methods,* 1983, vol. 65, 55-63